(19) Europäisches Patentamt
European Patent Office
Office européen des brevets

(11) EP 2 696 863 B1

(12) **FASCICULE DE BREVET EUROPEEN**

(45) Date de publication et mention de la délivrance du brevet:
**08.03.2017 Bulletin 2017/10**

(21) Numéro de dépôt: **12722400.4**

(22) Date de dépôt: **11.04.2012**

(51) Int Cl.:
*A61K 31/155* (2006.01) *A61K 31/4188* (2006.01)
*A61K 31/427* (2006.01) *A61K 31/43* (2006.01)
*A61K 31/431* (2006.01) *A61K 31/496* (2006.01)
*A61K 31/546* (2006.01) *A61K 31/57* (2006.01)
*A61K 31/665* (2006.01) *A61K 31/7016* (2006.01)
*A61K 31/7036* (2006.01) *A61K 45/06* (2006.01)
*A61P 31/04* (2006.01) *A61K 31/575* (2006.01)
*A61K 31/65* (2006.01) *A61K 38/12* (2006.01)

(86) Numéro de dépôt international:
**PCT/FR2012/050790**

(87) Numéro de publication internationale:
**WO 2012/140365 (18.10.2012 Gazette 2012/42)**

(54) **UTILISATION DE CALIXARENES ASSOCIES AVEC UN ANTIBIOTIQUE DANS LE TRAITEMENT DES INFECTIONS BACTERIENNES**

VERWENDUNG VON MIT EINEM ANTIBIOTIKUM ASSOZIIERTEN CALIXARENEN BEI DER BEHANDLUNG VON BAKTERIELLEN INFEKTIONEN

USE OF CALIXARENES ASSOCIATED WITH AN ANTIBIOTIC IN THE TREATMENT OF BACTERIAL INFECTIONS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **12.04.2011 FR 1153205**

(43) Date de publication de la demande:
**19.02.2014 Bulletin 2014/08**

(73) Titulaire: **Université de Lorraine**
**54052 Nancy Cedex (FR)**

(72) Inventeurs:
- **GRARE, Marion**
**F-31300 Toulouse (FR)**
- **DUVAL, Raphaël, Emmanuel**
**F-57170 Bioncourt (FR)**

(74) Mandataire: **Grosset-Fournier, Chantal Catherine et al**
**Grosset-Fournier & Demachy**
**54, rue Saint-Lazare**
**75009 Paris (FR)**

(56) Documents cités:
**WO-A2-95/19974 WO-A2-2006/042104**

- **GRARE MARION ET AL: "In vitro activity of para-guanidinoethylcalix[4] arene against susceptible and antibiotic-resistant Gram-negative and Gram-positive bacteria", JOURNAL OF ANTIMICROBIAL CHEMOTHERAPY, vol. 60, no. 3, septembre 2007 (2007-09), pages 575-581, XP002655308, ISSN: 0305-7453 cité dans la demande**
- **GRARE M ET AL: "Cationic compounds with activity against multidrug-resistant bacteria: interest of a new compound compared with two older antiseptics, hexamidine and chlorhexidine.", CLINICAL MICROBIOLOGY AND INFECTION : THE OFFICIAL PUBLICATION OF THE EUROPEAN SOCIETY OF CLINICAL MICROBIOLOGY AND INFECTIOUS DISEASES MAY 2010 LNKD- PUBMED:19456831, vol. 16, no. 5, mai 2010 (2010-05), pages 432-438, XP002655309, ISSN: 1469-0691 cité dans la demande**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

- GRARE M ET AL: "Cinetique d@?action du para-guanidinoethylcalix[4]arene, et evolution de la permeabilite membranaire", PATHOLOGIE ET BIOLOGIE, L'EXPANSION SCIENTIFIQUE FRANCAISE, PARIS, FR, vol. 58, no. 1, 1 février 2010 (2010-02-01), pages 46-51, XP026894782, ISSN: 0369-8114 [extrait le 2009-11-04] cité dans la demande
- MOURER M ET AL: "Functional organisation and gain of activity: The case of the antibacterial tetra-para-guanidinoethyl-calix[4]arene", BIOORGANIC & MEDICINAL CHEMISTRY LETTERS, PERGAMON, ELSEVIER SCIENCE, GB, vol. 16, no. 11, 1 juin 2006 (2006-06-01), pages 2960-2963, XP025106156, ISSN: 0960-894X, DOI: DOI:10.1016/J.BMCL.2006.02.072 [extrait le 2006-06-01] cité dans la demande

## Description

La présente invention concerne l'utilisation de calixarènes avec un antibiotique dans le traitement des infections bactériennes.

Dans le domaine de la santé publique, la lutte contre les infections bactériennes communautaires ou nosocomiales, est toujours un sujet d'actualité et d'inquiétudes. En effet, les bactéries sont les microorganismes le plus souvent responsables d'infections nosocomiales (IN), avec, par ordre de fréquence : *Escherichia coli* (24,7%), *Staphylococcus aureus* (18,9%), *Pseudomonas aeruginosa* (10%) et *Enterococcus spp.* (6%) (Enquête RAISIN 2006).

Certaines bactéries impliquées en clinique présentent une résistance voire, une multi-résistance aux antibiotiques et/ou antiseptiques utilisés en routine. On parle de BMR pour Bactéries Multi-Résistantes et de BTR pour Bactéries Toto- Résistantes. On peut citer par exemple les SARM (*Staphylococcus aureus* Résistants à la Méticilline, avec une résistance à l'ensemble des β-Lactamines), les Entérobactéries porteuses de BLSE (β-Lactamase à Spectre Etendu) ou encore les ERG (*Enterococcus* spp. Résistants aux Glycopeptides). A l'heure actuelle, 64% des *Staphylococcus aureus* isolés au cours d'IN sont résistants à la méticilline (Enquête RAISIN 2006). Le problème est que les bactéries sont souvent porteuses de plusieurs mécanismes de résistance, induisant une résistance à de nombreuses familles d'antibiotiques: β-lactamines, aminosides, fluoroquinolones ou macrolides... Par ailleurs, cette résistance aux antibiotiques, est souvent associée à une résistance aux antiseptiques, utilisés en milieu hospitalier, pour lutter contre la dissémination des infections nosocomiales.

La résistance d'une souche bactérienne vis-à-vis d'un antibiotique peut être une résistance naturelle (caractéristique de l'ensemble des souches d'une même espèce). Elle peut être aussi acquise (caractéristique de certaines souches au sein d'une espèce) ; elle résulte alors d'une modification du capital génétique de ces bactéries. Ce genre de modification génétique peut conférer à une souche bactérienne concernée un mécanisme de résistance à un antibiotique, à une famille d'antibiotiques ou à plusieurs familles d'antibiotiques.

La recherche fondamentale sur les mécanismes mis en jeu par les bactéries et les informations épidémiologiques suscitent actuellement des doutes sur la possibilité d'éradiquer les BMR dans l'avenir. Dès lors, il n'est plus certain que les antibiotiques disponibles actuellement permettent de contrôler durablement le problème. Si la mise à disposition de nouveaux antibiotiques a permis jusqu'à aujourd'hui de répondre à chaque forme de résistance bactérienne, cette démarche fait maintenant face à de nombreuses limites, puisque aucune nouvelle classe d'antibiotiques n'a été développée depuis vingt-cinq ans (Boucher *et al.* CID, 2009). Peu de nouveaux antibiotiques ont été commercialisés depuis le début des années 90. Parmi ces nouveaux antibiotiques, seuls le linézolide et la daptomycine, ont un mécanisme d'action innovant, mais sont réservés à des applications bien particulières et sont actifs uniquement sur les bactéries à Gram positif. En outre, ils présentent une toxicité importante (toxicité hématologique et médullaire pour le linézolide, pneumopathies à éosinophiles pour la daptomycine), ce qui a restreint leur usage.

Cependant, peu de temps après leur commercialisation, des résistances sont apparues. Ainsi, à titre d'exemple, on peut citer les cas du linézolide, de la daptomycine, de la quinupristine-dalfopristine, ou de la tigécycline, y compris chez des bactéries initialement BMR.

En mettant en jeu un concept innovant liant chimie supramoléculaire avec ciblage et désorganisation de l'enveloppe bactérienne, une nouvelle famille de composés antibactériens, en particulier le para-guanidinoéthylcalix[4]arène, désigné ci-après Cx1, a été développée récemment. Cette famille de composés présente des propriétés antibactériennes vis-à-vis de différentes bactéries impliquées dans les infections nosocomiales et/ou communautaires.

La publication de Grare et al. (J. Antimicrob. Chemother. 60 (2007), 575-581) décrit que le Cx1 possède une activité antibactérienne sur les bactéries résistantes ou non aux antibiotiques.

Dans la publication de Grare et al. (Clin. Microbiol. Infect. 16 (2010), 432-438), l'activité antibactérienne du Cx1 est comparée à celle de l'hexamidine et de la chlorhexidine, deux antiseptiques, d'utilisation très courante en thérapeutique humaine, sur toute une série d'isolats cliniques : MDR (« multidrug resistant »), XDR (« extended drug resistant »), voire PDR (« pan-drug resistant »).

L'article de Grare et al. (Pathologie Biologie 58 (2010), 46-51) décrit que le Cx1, en tant qu'antibactérien cationique, interagit avec la paroi bactérienne, entraînant au final une perte d'intégrité membranaire.

Néanmoins, face à la menace d'apparition de bactéries PDR, l'urgence absolue reste de pouvoir disposer, de nouveaux composés antibactériens possédant des mécanismes d'action innovants pour traiter les patients infectés par ce type de bactéries ; et/ou de moyens nouveaux permettant de rendre le traitement par des antibiotiques utilisés normalement en thérapeutique anti-infectieuse, à nouveau accessibles à ces patients.

Un aspect de la présente invention est de fournir de nouveaux produits antibactériens.

Un autre aspect de l'invention est de fournir de nouvelles compositions d'antibactériens associant des calixarènes et des antibiotiques.

La présente invention repose sur un fait inattendu constaté par les Inventeurs, lors de l'évaluation de l'activité antibactérienne du para-guanidinoéthylcalix[4]arène, désigné ci-après Cx1. Cette molécule permet de diminuer la CMI (Concentration Minimale Inhibitrice) d'un antibiotique vis-à-vis duquel une souche bactérienne présente une résistance.

En d'autre terme, d'un côté, le Cx1 permet de conférer *de novo* un certain niveau de sensibilité vis-à-vis d'(un) antibiotique(s) chez une souche bactérienne présentant une résistance acquise audit(s) antibiotique(s), et d'un autre côté, le Cx1 est aussi capable de conférer une sensibilité vis-à-vis d'(un) antibiotique(s) chez une souche bactérienne présentant une résistance naturelle audit(s) antibiotique(s).

Concrètement, dans le cadre clinique, le traitement du Cx1 en association avec le traitement d'au moins un antibiotique permet de diminuer la dose de ce dernier dans le cadre du traitement d'une infection par une bactérie résistante audit antibiotique, et/ou de rendre le traitement par ledit antibiotique efficace aux patients présentant une infection à au moins une souche bactérienne résistante audit antibiotique.

La présente invention propose un produit comprennant au moins un antibiotique donné, et un calixarène représenté par la formule I suivante :

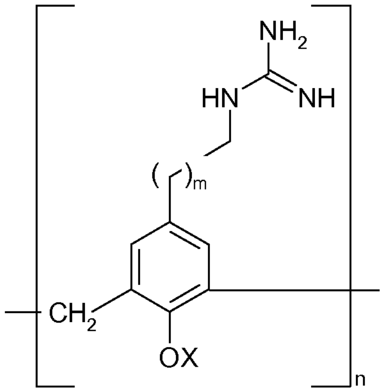

Formule I

,

dans laquelle :

(i) n = un entier de 4 à 16,
(ii) m= un entier de 1 à 10,
(iii) X est choisi parmi :

- un hydrogène,
- un groupement alkyl, le nombre de carbones étant de 1 à 20, notamment de 1 à 10,
- un halogène choisi parmi Cl, Br, I, ou
- un groupement amphiphile choisi parmi un groupe anionique, tels que les carboxylates $-RCO_2^-$, les sulfates $-RSO_4^-$, les sulfonates $-RSO_3^-$, un groupe cationique, tel que $RNH_3^+$, dans lesquels R est un groupement alkyl, le nombre de carbones étant de 1 à 20, notamment de 1 à 10,

pour son utilisation comme médicament.

Dans un mode de réalisation particulier, le produit selon l'invention comprend un antibiotique donné et un calixarène représenté par la formule I, dans laquelle n=4, ledit calixarène représenté par la formule I(1) suivante :

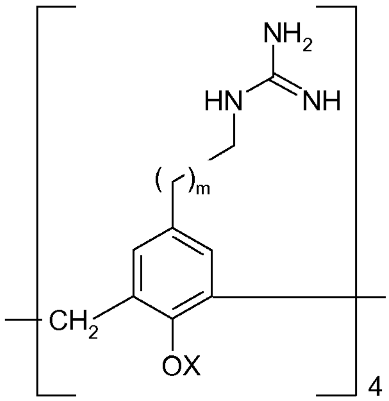

Formule I(1)

m et X ayant les significations indiquées ci-dessus.

Dans un autre mode de réalisation particulier, le produit selon l'invention comprend un antibiotique donné et un calixarène représenté par la formule I, dans laquelle m=1, ledit calixarène représenté par la formule I(2) suivante :

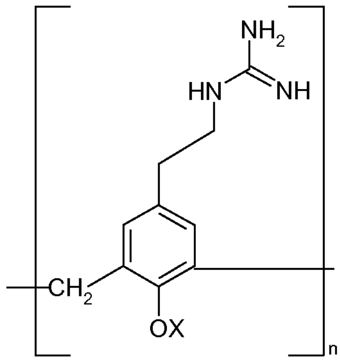

Formule I(2)

n et X ayant les significations indiquées ci-dessus.

Selon un autre mode de réalisation particulier, le produit selon l'invention comprend un antibiotique donné et un calixarène représenté par la formule I, dans laquelle X est un hydrogène, ledit calixarène représenté par la formule I(3) suivante :

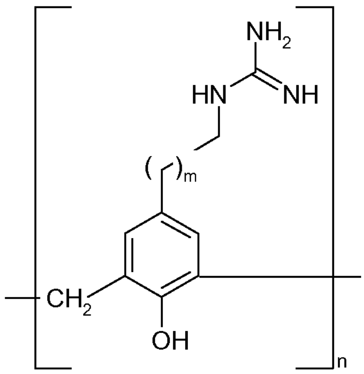

Formule I(3)

m et n ayant les significations indiquées ci-dessus.

Dans un mode de réalisation avantageux, la présente invention concerne un produit pour son utilisation comme médicament, ledit produit comprenant un antibiotique donné et un calixarène représenté par la formule I, dans laquelle n=4, m=1 et X est un hydrogène, ledit calixarène représenté par la formule II suivante :

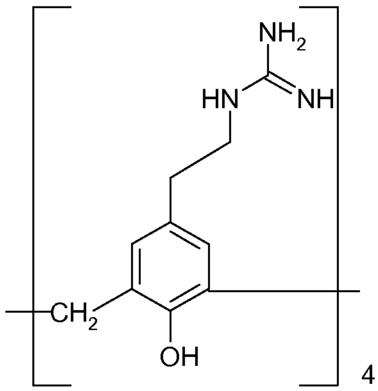

Formule II

La molécule représentée par la formule II est le para-guanidinoéthylcalix[4]arène, désigné par Cx1 dans la présente invention.

La structure tridimensionnelle de la susdite molécule est illustrée ci-après.

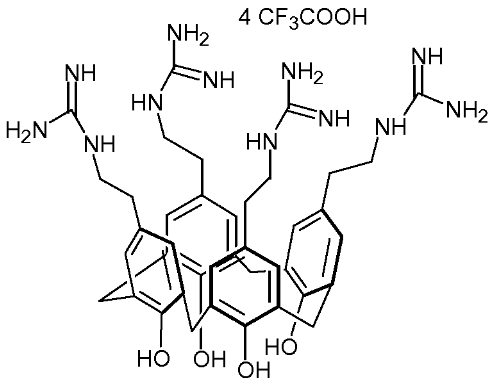

Le Cx1 peut être synthétisé selon le procédé décrit dans Mourer et al. (Bioorganic & Médicinal Chemistry Letter 16 (2006) 2960-2963).

Le calixarène selon l'invention peut être tel que décrit ci-dessus, ou un sel d'acide physiologiquement acceptable dérivé d'un composé de formule (I) tel qu'un chlorhydrate, un formiate, un trifluoroacétate ou un oxalate (HOOCCOOH).

L'expression « sel d'acide physiologiquement acceptable» signifie un dérivé d'un composé de formule I, obtenu par réaction d'un acide inorganique ou d'un acide organique, sur un composé de formule I.

Des exemples d'acides inorganiques permettant l'obtention de sels physiologiquement acceptables incluent sans être limités à ceux-ci, l'acide chlorhydrique, l'acide bromhydrique, l'acide nitrique, l'acide carbonique, l'acide formique, l'acide monohydrogénocarbonique, l'acide phosphorique, l'acide monohydrogénophosphorique, l'acide dihydrogéno-phosphorique, l'acide perchlorique, l'acide sulfurique, l'acide monohydrogénosulfurique, l'acide iodhydrique.

Des exemples d'acides organiques permettant l'obtention de sels physiologiquement acceptables incluent sans être limités à ceux-ci, l'acide acétique, l'acide lactique, l'acide propionique, l'acide butyrique, l'acide isobutyrique, l'acide palmique, l'acide maléique, l'acide glutamique, l'acide hydroxymaléique, l'acide malonique, l'acide benzoïque, l'acide succinique, l'acide glycolique, l'acide subérique, l'acide fumarique, l'acide mandélique, l'acide phthalique, l'acide salicylique, l'acide benzènesulfonique, l'acide *p*-toluènesulfonique, l'acide citrique, l'acide tartrique, l'acide méthanesulfonique, l'acide hydroxynaphthoïque.

Les sels d'acides aminés, tels que les arginates et leurs équivalents sont également inclus ainsi que les sels d'acides organiques tels que l'acide glucuronique ou l'acide galacturonique et leurs équivalents (voir, par exemple, Berge et al., "Pharmaceutical Salts", Journal of Pharmaceutical Science, 1977, 66, 1-19).

L'antibiotique donné mis en oeuvre dans le susdit produit pour son utilisation comme médicament selon l'invention peut être choisi parmi les β- lactamines, les aminosides, les fluoroquinolones, la fosfomycine, la colimycine, la rifampicine, la tigécycline, ou l'acide fusidique, et plus particulièrement dans le groupe comprenant l'imipénème, la pipéracilline-tazobactam, la pénicilline G, le céfotaxime, la ceftazidime, la tobramycine, la gentamicine, la ciprofloxacine, la rifampicine, la fosfomycine, la colimycine, la streptomycine, la ticarcilline-acide clavulanique, la tigécycline ou l'acide fusidique.

Dans un mode de réalisation particulièrement avantageux, le produit selon l'invention comprend un calixarène représenté par la formule II (Cx1) et la tigécycline.

Dans un autre mode de réalisation particulièrement avantageux, le produit selon l'invention comprend un calixarène représenté par la formule II (Cx1) et l'acide fusidique.

Dans un autre mode de réalisation particulièrement avantageux, le produit selon l'invention comprend un calixarène représenté par la formule II (Cx1) et la fosfomycine.

Dans un autre mode de réalisation particulièrement avantageux, le produit selon l'invention comprend un calixarène représenté par la formule II (Cx1) et la pénicilline.

Dans un autre mode de réalisation particulièrement avantageux, le produit selon l'invention comprend un calixarène représenté par la formule II (Cx1) et l'imipénème.

Dans un autre mode de réalisation particulièrement avantageux, le produit selon l'invention comprend un calixarène représenté par la formule II (Cx1) et la gentamicine.

Dans un autre mode de réalisation particulièrement avantageux, le produit selon l'invention comprend un calixarène représenté par la formule II (Cx1) et le céfotaxime.

Dans un autre mode de réalisation particulièrement avantageux, le produit selon l'invention comprend un calixarène représenté par la formule II (Cx1) et la rifampicine.

Dans un autre mode de réalisation particulièrement avantageux, le produit selon l'invention comprend un calixarène représenté par la formule II (Cx1) et la pipéracilline-tazobactam.

Dans un autre mode de réalisation particulièrement avantageux, le produit selon l'invention comprend un

calixarène représenté par la formule II (Cx1) et la ciprofloxacine.

Dans un autre mode de réalisation particulièrement avantageux, le produit selon l'invention comprend un calixarène représenté par la formule II (Cx1) et la ceftazidime.

Dans un autre mode de réalisation particulièrement avantageux, le produit selon l'invention comprend un calixarène représenté par la formule II (Cx1) et la colimycine.

Dans un autre mode de réalisation particulièrement avantageux, le produit selon l'invention comprend un calixarène représenté par la formule II (Cx1) et la ticarcilline-acide clavulanique.

Dans un autre mode de réalisation particulièrement avantageux, le produit selon l'invention comprend un calixarène représenté par la formule II (Cx1) et la tobramycine.

Dans une telle composition selon l'invention, le calixarène représenté par la formule II (Cx1) et un antibiotique donné peuvent être physiquement mélangés ensemble dans un produit unitaire final.

Le calixarène représenté par la formule II (Cx1) et un tel antibiotique donné peuvent être aussi présents sous forme d'un même produit unitaire final, mais physiquement séparés. Par exemple, le Cx1 et l'antibiotique donné peuvent être présents respectivement dans deux compartiments séparés d'une gélule.

Un autre aspect de l'invention concerne un produit tel que décrit ci-dessus pour son utilisation comme médicament dans le traitement de pathologies impliquant une souche bactérienne présentant une résistance à au moins un antibiotique déterminé.

Dans un mode de réalisation avantageux, l'invention concerne un produit tel que décrit ci-dessus pour son utilisation dans le traitement de pathologies impliquant une souche bactérienne résistante parmi *Escherichia coli, Pseudomonas aeruginosa,* et *Staphylococcus aureus,* plus particulièrement une souche bactérienne résistante choisie parmi :

- une souche de *Staphylococcus aureus* de phénotype sauvage
- une souche de *Staphylococcus aureus* Résistant à la Méticilline (SARM) sans résistance associée,
- une souche de SARM présentant une résistance aux aminosides et aux fluoroquinolones,
- une souche de SARM présentant une résistance aux aminosides, aux fluoroquinolones, aux macrolides-lincosamides-synergystines et à l'ofloxacine,
- une souche *d'Escherichia coli* de phénotype sauvage
- une souche *d'Escherichia coli* productrice de pénicillinase sans résistance associée,
- une souche *d'Escherichia coli* productrice de BLSE (β-Lactamase à Spectre Etendu), présentant une résistance associée aux aminosides, à la rifampicine et à l'association triméthoprime-sulfaméthoxazo le,
- une souche *d'Escherichia coli* hyperproductrice de céphalosporinase, présentant une résistance associée aux aminosides, aux quinolones et à l'association triméthoprime-sulfaméthoxazole,
- une souche de *Pseudomonas aeruginosa* de phénotype sauvage
- une souche de *Pseudomonas aeruginosa* présentant une résistance aux β-lactamines, à l'association triméthoprime-sulfaméthoxazole et à la fosfomycine,
- une souche de *Pseudomonas aeruginosa* présentant une résistance aux β-lactamines (y compris les carbapénèmes), aux aminosides, à l'association triméthoprime-sulfaméthoxazole et à la ciprofloxacine,
- une souche muqueuse de *Pseudomonas aeruginosa* présentant une résistance à la rifampicine et à l'association triméthoprime-sulfaméthoxazole.

Le produit selon l'invention est notamment utilisé dans le traitement des pathologies impliquant une souche de bactéries présentant une résistance, notamment les infections nosocomiales et/ou communautaires, telles que les infections abdominales, les infections digestives, les infections urinaires, les infections respiratoires, les infections neuro-méningées, les infections de la sphère oro-pharyngée, les infections génitales, les endocardites, les infections de la peau et des tissus mous, les infections ostéo-articulaires, les infections oculaires, les septicémies ou bactériémies, plus particulièrement dans le traitement des pathologies citées ci-dessous dans le tableau 1.

Tableau 1

| | |
|---|---|
| infections abdominales | péritonite, appendicite... |
| infections digestives | diarrhées toxi-infection alimentaire collectives, diarrhées post-antibiothérapie... |
| infections urinaires | cystites, pyélonéphrites, prostatites... |
| infections respiratoires | bronchites, pneumonies, pneumopathies, abcès... |
| infections neuro-méningées | méningites bactériennes, abcès cérébraux... |

(suite)

| | |
|---|---|
| infections de la sphère oro-pharyngée | sinusites, otites, angines, phlegmons, épiglottites... |
| infections génitales | vulvite, vaginite/vaginose, cervicite, salpingite... |
| infections de la peau et des tissus mous | furonculose, abcès, escarre, pied diabétique ... |
| infections oculaires | conjonctivites, kératites, endophtalmies... |
| autres infections | septicémies ou bactériémies... |

Dans un mode de réalisation particulièrement avantageux, l'invention concerne un produit comprenant :

- un calixarène représenté par la formule II, et
- un antibiotique donné choisi parmi la tigécycline, l'acide fusidique ou la fosfomycine pour son utilisation dans le traitement de pathologies impliquant la souche de SARM sans résistance associée.

Dans un autre mode de réalisation particulièrement avantageux, l'invention concerne un produit comprenant :

- un calixarène représenté par la formule II, et
- un antibiotique donné choisi parmi la pénicilline G, la tigécycline, l'acide fusidique ou la fosfomycine pour son utilisation dans le traitement de pathologies impliquant la souche de SARM présentant une résistance aux aminosides et aux fluoroquinolones.

Dans un autre mode de réalisation particulièrement avantageux, l'invention concerne un produit comprenant :

- un calixarène représenté par la formule II, et
- un antibiotique donné choisi parmi la pénicilline G, l'imipénème, la gentamicine, la tigécycline, l'acide fusidique ou la fosfomycine pour son utilisation dans le traitement de pathologies impliquant la souche de SARM présentant une résistance aux macrolides, aux fluoroquinolones,aux macrolides-lincosamides-synergistines et à l'ofloxacine.

Dans un autre mode de réalisation particulièrement avantageux, l'invention concerne un produit comprenant :

- un calixarène représenté par la formule II, et
- un antibiotique donné choisi parmi la tigécycline, l'acide fusidique ou la fosfomycine pour son utilisation dans le traitement de pathologies impliquant la souche *Staphylococcus aureus* de phénotype sauvage.

On entend « une souche de *Staphylococcus aureus* de phénotype sauvage », une souche de *Staphylococcus aureus* qui ne possède pas de mécanismes de résistance acquise aux antibiotiques (uniquement des résistances naturelles).

Dans un autre mode de réalisation particulièrement avantageux, l'invention concerne un produit comprenant :

- un calixarène représenté par la formule II, et
- un antibiotique donné choisi parmi le céfotaxime, la gentamicine ou la rifampicine pour son utilisation dans le traitement de pathologies impliquant la souche *d'Escherichia coli* productrice de BLSE présentant une résistance associée aux aminosides, à la rifampicine et à l'association triméthoprime-sulfaméthoxazole.

Dans un autre mode de réalisation particulièrement avantageux, l'invention concerne un produit comprenant :

- un calixarène représenté par la formule II, et
- un antibiotique donné choisi parmi la gentamicine ou la rifampicine pour son utilisation dans le traitement de pathologies impliquant la souche d'*Escherichia coli* productrice de pénicillinase sans résistance associée, ou la souche *d'Escherichia coli* hyperproductrice de céphalosporinase présentant une résistance associée aux aminosides, aux quinolones et à l'association triméthoprime-sulfaméthoxazole.

Dans un autre mode de réalisation particulièrement avantageux, l'invention concerne un produit comprenant :

- un calixarène représenté par la formule II, et
- un antibiotique donné choisi parmi la gentamicine, la tobramycine, ou la rifampicine son utilisation dans le traitement de pathologies impliquant une souche d'*Escherichia coli* de phénotype sauvage.

On entend « une souche *d'Escherichia coli* de phénotype sauvage », une souche d'*E. coli* qui ne possède pas de mécanismes de résistance acquise aux antibiotiques (uniquement des résistances naturelles).

Dans un autre mode de réalisation particulièrement avantageux, l'invention concerne un produit comprenant :

- un calixarène représenté par la formule II, et
- un antibiotique donné choisi parmi la pipéracilline-tazobactam, la rifampicine, la tobramycine pour son utilisation dans le traitement de pathologies impliquant une souche de *Pseudomonas aeruginosa* présentant une résistance aux β-lactamines, à l'association triméthoprime-sulfaméthoxazole et à la fosfomycine.

Dans un autre mode de réalisation particulièrement avantageux, l'invention concerne un produit comprenant :

- un calixarène représenté par la formule II, et
- un antibiotique donné choisi parmi la ceftazidime, la rifampicine, la colimycine, la fosfomycine pour son utilisation dans le traitement de pathologies impliquant une souche de *Pseudomonas aeruginosa* présentant une résistance aux β-lactamines (y compris les carbapénèmes), aux aminosides, à l'association triméthoprime-sulfaméthoxazole et à la ciprofloxacine.

Dans un autre mode de réalisation particulièrement avantageux, l'invention concerne un produit comprenant :

- un calixarène représenté par la formule II, et
- un antibiotique donné choisi parmi la pipéracilline-tazobactam, l'imipénème, la rifampicine, la colimycine, la fosfomycine, la tobramycine et la ciprofloxacine pour son utilisation dans le traitement de pathologies impliquant une souche muqueuse de *Pseudomonas aeruginosa* présentant une résistance à la rifampicine et à l'association triméthoprime-sulfaméthoxazo le.

Dans un autre mode de réalisation particulièrement avantageux, l'invention concerne un produit comprenant :

- un calixarène représenté par la formule II, et
- un antibiotique donné choisi parmi la pipéracilline-tazobactam, la ceftazidime, la tobramycine, la ciprofloxacine, la rifampicine, la fosfomycine ou la ticarcilline-acide clavulanique pour son utilisation dans le traitement de pathologies impliquant une souche de *Pseudomonas aeruginosa* de phénotype sauvage.

On entend « une souche de *Pseudomonas aeruginosa* de phénotype sauvage », une souche de *P. aeruginosa* qui ne possède pas de mécanismes de résistance acquise aux antibiotiques (uniquement des résistances naturelles).

La présente invention concerne également une composition pharmaceutique comprenant au moins un produit tel que décrit ci-dessus comme substance active en association avec un véhicule pharmaceuticalement acceptable.

Diverses formulations sont possibles pour les dites compositions pharmaceutiques : sous forme de gélule, comprimé, poudre, crème, lotion, solution aqueuse ou hydro-alcoolique, collutoire, collyre, lait, mousse, gel, spray ou poudre par exemple.

Ladite composition pharmaceutique peut être administrée par voie orale, parentérale, ou topique.

Dans une telle composition pharmaceutique selon l'invention, l'homme du métier sait que la dose d'administration unitaire du Cx1 dépend de la nature des bactéries à traiter, mais également de la dose unitaire d'un antibiotique donné.

La dose d'administration unitaire d'un antibiotique donné classique est connue de l'homme du métier.

Un autre aspect de la présente invention a pour objet de fournir un produit de combinaison pour une utilisation simultanée, séparée ou étalée dans le temps en thérapie de pathologies impliquant au moins une souche bactérienne présentant une résistance.

Ledit produit de combinaison contient :

- un antibiotique donné choisi parmi : les β-lactamines, les aminosides, les fluoroquinolones, la fosfomycine, la colimycine, la rifampicine, la tigécycline, ou l'acide fusidique, et
- un calixarène représenté par la formule I suivante :

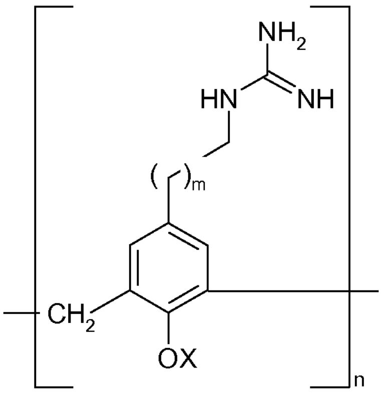

Formule I

,

dans laquelle :

(i) n = un entier de 4 à 16,
(ii) m= un entier de 1 à 10,
(iii) X est choisi parmi :

- un hydrogène,
- un groupement alkyl, le nombre de carbones étant de 1 à 20, notamment de 1 à 10,
- un halogène choisi parmi Cl, Br, I, ou
- un groupement amphiphile choisi parmi un groupe anionique, tels que les carboxylates $-RCO_2^-$, les sulfates $-RSO_4^-$, les sulfonates $-RSO_3^-$, un groupe cationique, tel que $RNH_3^+$, dans lesquels R est un groupement alkyl, le nombre de carbones étant de 1 à 20, notamment de 1 à 10,

comme produit de combinaison, pour une utilisation simultanée, séparée ou étalée dans le temps pour le traitement de pathologies impliquant au moins une souche bactérienne présentant une résistance acquise à au moins un antibiotique déterminé, telles que les infections abdominales, les infections digestives, les infections urinaires, les infections respiratoires, les infections neuro-méningées, les infections de la sphère oro-pharyngée, les infections génitales, les endocardites, les infections de la peau et des tissus mous, les infections ostéo-articulaires, les infections oculaires, les septicémies ou bactériémies, plus particulièrement les pathologies citées ci-dessus dans le tableau 1.

Dans un tel produit de combinaison selon l'invention, le calixarène représenté par la formule I et l'antibiotique donné sont présents physiquement séparés dans un produit final. Le calixarène et l'antibiotique donné peuvent être administrés aux patients simultanément, séparément ou selon un ordre étalé dans le temps, selon la prescription.

Dans un mode de réalisation particulier, l'invention concerne un produit de combinaison pour son utilisation telle que décrite ci-dessus, dans lequel le calixarène correspond au calixarène représenté par la formule II suivante :

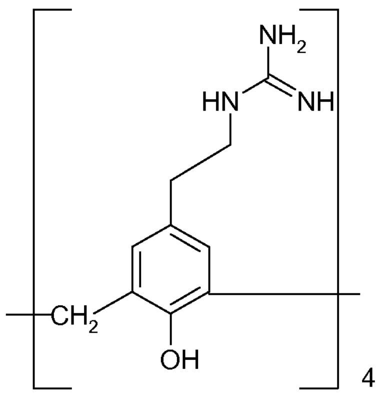

Formule II

La molécule représentée par la formule II est le para-guanidinoéthylcalix[4]rène, désigné par Cx1 dans la présente demande.

Dans un autre mode de réalisation avantageux du produit de combinaison de l'invention pour son utilisation telle décrite ci-dessus, l'antibiotique donné est choisi parmi l'imipénème, la pipéracilline-tazobactam, la pénicilline G, le

céfotaxime, la ceftazidime, la tobramycine, la gentamicine, la ciprofloxacine, la rifampicine, la fosfomycine, la colimycine, la streptomycine, la ticarcilline-acide clavulanique, la tigécycline ou l'acide fusidique.

Dans un autre mode de réalisation particulier, l'invention concerne un produit de combinaison pour une utilisation simultanée, séparée ou étalée dans le temps en thérapie de pathologies impliquant au moins une souche bactérienne résistante appartenant à une espèce choisie parmi *Escherichia coli, Pseudomonas aeruginosa,* et *Staphylococcus aureus.*

Dans un autre mode de réalisation particulier, le produit de combinaison selon l'invention est destiné pour une utilisation simultanée, séparée ou étalée dans le temps en thérapie de pathologies impliquant au moins une souche bactérienne résistante choisie parmi :

- une souche de *Staphylococcus aureus* de phénotype sauvage
- une souche de *Staphylococcus aureus* Résistant à la Méticilline (SARM) sans résistance associée,
- une souche de SARM présentant une résistance aux aminosides et aux fluoroquinolones,
- une souche de SARM présentant une résistance aux aminosides, aux fluoroquinolones, aux macrolides-lincosamides-synergystines et à l'ofloxacine,
- une souche *d'Escherichia coli* de phénotype sauvage
- une souche *d'Escherichia coli* productrice de pénicillinase sans résistance associée,
- une souche *d'Escherichia coli* productrice de BLSE (β-Lactamase à Spectre Etendu), présentant une résistance associée aux aminosides, à la rifampicine et à l'association triméthoprime-sulfaméthoxazo le,
- une souche d'*Escherichia coli* hyperproductrice de céphalosporinase présentant une résistance associée aux aminosides, aux quinolones et à l'association triméthoprime-sulfaméthoxazole,
- une souche de *Pseudomonas aeruginosa* de phénotype sauvage
- une souche de *Pseudomonas aeruginosa* présentant une résistance aux β-lactamines, à l'association triméthoprime-sulfaméthoxazole et à la fosfomycine,
- une souche de *Pseudomonas aeruginosa* présentant une résistance aux β-lactamines (y compris les carbapénèmes), aux aminosides, à l'association triméthoprime-sulfaméthoxazole et à la ciprofloxacine,
- une souche muqueuse de *Pseudomonas aeruginosa* présentant une résistance à la rifampicine et à l'association triméthoprime-sulfaméthoxazole.

Dans un mode de réalisation particulier, l'invention concerne un produit contenant le le calixarène de formule II (Cx1) et un antibiotique donné choisi parmi la tigécycline, l'acide fusidique ou la fosfomycine, comme produit de combinaison, pour une utilisation simultanée, séparée ou étalée dans le temps en thérapie de pathologies impliquant la souche de SARM sans résistance associée.

Dans un autre mode de réalisation particulier, l'invention concerne un produit contenant le calixarène de formule II (Cx1) et un antibiotique donné choisi parmi la pénicilline G, la tigéncyline, l'acide fusidique ou la fosfomycine, comme produit de combinaison pour une utilisation simultanée, séparée ou étalée dans le temps en thérapie de pathologies impliquant la souche de SARM présentant une résistance aux aminosides et aux fluoroquinolones.

Dans un autre mode de réalisation particulier, l'invention concerne un produit contenant le calixarène de formule II (Cx1) et un antibiotique donné choisi parmi la pénicilline G, l'imipénème, la gentamicine, la tigécycline, l'acide fusidique ou la fosfomycine, comme produit de combinaison pour son utilisation simultanée, séparée ou étalée dans le temps en thérapie de pathologies impliquant la souche de SARM présentant une résistance aux aminosides, aux fluoroquinolones, aux macrolides-lincosamides-synergistines
et à l'ofloxacine.

Dans un autre mode de réalisation particulier, l'invention concerne un produit contenant le calixarène de formule II (Cx1) et un antibiotique donné choisi parmi la tigécycline, l'acide fusidique ou la fosfomycine, comme produit de combinaison pour son utilisation simultanée, séparée ou étalée dans le temps en thérapie de pathologies impliquant la souche *Staphylococcus aureus* de phénotype sauvage.

Dans un autre mode de réalisation particulier, l'invention concerne un produit contenant le calixarène de formule II (Cx1) et un antibiotique donné choisi parmi le céfotaxime, la gentamicine ou la rifampicine, comme produit de combinaison pour son utilisation simultanée, séparée ou étalée dans le temps en thérapie de pathologies impliquant la souche *d'Escherichia coli* productrice de BLSE présentant une résistance associée aux aminosides, à la rifampicine et à l'association triméthoprime-sulfaméthoxazole.

Dans un autre mode de réalisation particulier, l'invention concerne un produit contenant le calixarène de formule II (Cx1) et un antibiotique donné choisi parmi la gentamicine ou la rifampicine, comme produit de combinaison pour son utilisation simultanée, séparée ou étalée dans le temps en thérapie de pathologies impliquant la souche *d'Escherichia coli* productrice de pénicillinase sans résistance associée, ou la souche d'*Escherichia coli* hyperproductrice de céphalosporinase présentant une résistance associée aux aminosides, aux quinolones et à l'association triméthoprime-sulfaméthoxazole.

Dans un autre mode de réalisation particulier, l'invention concerne un produit contenant le calixarène de formule II (Cx1) et un antibiotique donné choisi parmi la gentamicine, la tobramycine, ou la rifampicine, comme produit de combinaison, pour son utilisation simultanée, séparée ou étalée dans le temps en thérapie de pathologies impliquant la souche *d'Escherichia coli* de phénotype sauvage.

Dans un autre mode de réalisation particulier, l'invention concerne un produit contenant le calixarène de formule II (Cx1) et un antibiotique donné choisi parmi la pipéracilline-tazobactam, la rifampicine, la tobramycine, comme produit de combinaison, pour son utilisation simultanée, séparée ou étalée dans le temps en thérapie de pathologies impliquant une souche de *Pseudomonas aeruginosa* présentant une résistance aux β-lactamines, à l'association triméthoprime-sulfaméthoxazole et à la fosfomycine.

Dans un autre mode de réalisation particulier, l'invention concerne un produit contenant le calixarène de formule II (Cx1) et un antibiotique donné choisi parmi la ceftazidime, la rifampicine, la colimycine, la fosfomycine pour son utilisation simultanée, séparée ou étalée dans le temps en thérapie de pathologies impliquant une souche *Pseudomonas aeruginosa* présentant une résistance aux β-lactamines (y compris les carbapénèmes), aux aminosides, à l'association triméthoprime-sulfaméthoxazole et à la ciprofloxacine.

Dans un autre mode de réalisation particulier, l'invention concerne un produit contenant le calixarène de formule II (Cx1) et un antibiotique donné choisi parmi la pipéracilline-tazobactam, l'imipénème, la rifampicine, la colimycine, la fosfomycine, la tobramycine et la ciprofloxacine, comme produit de combinaison, pour son utilisation simultanée, séparée ou étalée dans le temps en thérapie de pathologies impliquant une souche muqueuse de *Pseudomonas aeruginosa* présentant une résistance à la rifampicine et à l'association triméthoprime-sulfaméthoxazo le.

Dans un autre mode de réalisation particulier, l'invention concerne un produit contenant le calixarène de formule II (Cx1) et un antibiotique donné choisi parmi la pipéracilline-tazobactam, la ceftazidime, la tobramycine, la ciprofloxacine, la rifampicine, la fosfomycine ou la ticarcilline-acide clavulanique, comme produit de combinaison, pour son utilisation simultanée, séparée ou étalée dans le temps en thérapie de pathologies impliquant une souche de *Pseudomonas aeruginosa* de phénotype sauvage.

Les figures et les exemples ci-dessous permettant d'illustrer à titre d'exemple la présente invention, ne peuvent être interprétés en aucun cas la limitation de la portée de l'invention.

**Figures**

Figure 1 : la figure 1 représente une microplaque préparée pour mesurer la sensibilité d'une souche bactérienne vis-à-vis d'une solution contenant un antibiotique et le Cx1 respectivement dans une proportion des concentrations déterminées. Les colonnes 1 et 12 ne contiennent que du milieu témoin. Les colonnes 2 et 11 contiennent du milieu témoin et des bactéries, mais sans antibiotique ni le Cx1. Les colonnes 3 à 10 contiennent les bactéries, le Cx1 en concentration décroissante et un antibiotique en concentration croissante.
Figure 2A : la figure 2A illustre une additivité entre le Cx1 et un autre antibiotique.
Figure 2B : la figure 2B illustre une indifférence entre le Cx1 et un autre antibiotique.
Figure 2C : la figure 2C illustre une synergie entre le Cx1 et un autre antibiotique.
Figure 2D : la figure 2D illustre un antagonisme entre le Cx1 et un autre antibiotique.

**1. Matériel et Méthode**

**1.1 Matériel et réactif**

- Seringue de 10, 20 ou 50 mL
- Filtre 0,22 μm (filtres Millex®GP, 0,22 μm, Millipore, France)
- Tubes Falcon 15 et 50 mL
- Plaques de 96 puits (Greiner, 650161)
- Géloses Mueller Hinton (MHA) (Difco, 225250)
- Bouillons Mueller Hinton (MHB) (Difco, 275730)
- Eau distillée stérile

Solution de la drogue à tester : Cx1 (M = 1221,11 g/mol) fournie par le Pr. Regnouf de Vains sous forme de poudre blanche, reprise en eau distillée stérile et filtrée sur 0,22 μm pour obtenir une solution stérile à 10-2 mol/L. Nous nous sommes procuré les antibiotiques commerciaux par les fabricants, sous forme de poudre stérile, prête à l'emploi.

### 1.2 Souches bactériennes

Trois souches de référence ont été utilisées, correspondant à celles étudiées pour les CMI et les CMB (Concentration Minimale Bactéricide) : *Escherichia coli* ATCC 25922, *Staphylococcus aureus* ATCC 29213, *Pseudomonas aeruginosa* ATCC 27853. Pour chacune de ces souches ont été choisis 3 isolats cliniques correspondant, présentant divers profils de résistance aux antibiotiques classiquement utilisés en routine :

- EcR1, EcR2, EcR3;
- SaR1, SaR3, SaR4;
- PaR2, PaR3, PaR5.

Les profils de sensibilité aux antibiotiques de ces isolats cliniques sont figurés Annexe 1.
Les fluctuations relatives au type de résistance associée pour certaines souches sont dues à l'évolution des recommandations françaises (CA-SFM), européennes (EUCAST) et américaines (CLSI) en matière de catégorisation des souches bactériennes.

### 1.3. Mode opératoire : Technique de l'échiquier

#### J-1 : Mise en culture des bactéries sur gélose MHA (Mueller Hinton Agar)

Incuber 24 h à 35°C.

#### J0 : Ensemencement d'un bouillon MHB (Mueller Hinton Broth)

Prélever une colonie « moyenne » sur la gélose J-1 et ensemencer 5 mL de bouillon MHB.
Incuber 24 h à 35°C.

#### J1 : Préparation des plaques de 96 puits

Préparation de l'inoculum bactérien :

La pureté des souches est vérifiée par l'absence de contaminants sur la gélose MHA ensemencée parallèlement au bouillon, et par réalisation d'une coloration de Gram.
La suspension bactérienne est transférée dans un tube Falcon de 15 mL, centrifugée 10 min à 4500g puis le culot est remis en suspension dans 1 mL d'eau distillée stérile. Les dilutions adéquates sont ensuite réalisées afin d'obtenir un inoculum bactérien entre $5.10^5$ et $5.10^6$ UFC/mL.

Préparer les solutions : 1) Antibiotique (ATB) à tester, 2) Cx1

Les CMI des antibiotiques ont été déterminées préalablement pour chaque souche, par méthode de microdilution en milieu liquide (CLSI (Clinical and Laboratory Standards Institute), 2003].
Les dilutions adéquates sont réalisées afin d'obtenir une solution de concentration équivalente à 32 fois la CMI de l'ATB à tester, en milieu MH (Mueller-Hinton). Puis nous avons procédé à une série de dilutions d'ordre 2 en milieu MH afin d'obtenir les concentrations suivantes : 16, 8, 4, 2, 1, 0,5 et 0,25 fois la CMI (tubes Falcon de 15 mL). Le même mode opératoire est suivi pour le Cx1. Cela permet d'obtenir une gamme de concentration de 8 à 0,06 fois la CMI dans la microplaque pour les deux molécules (dilution au demi avec addition de la 2ème molécule, puis nouvelle dilution au ½ après ajout de la suspension bactérienne). On obtient ainsi 64 combinaisons ATB/Cx1.
Pour chaque plaque, 1 mL de solution de chaque dilution est nécessaire.

Préparation des microplaques

Le volume final contenu dans chacun des puits doit être de 100 $\mu$L. Deux témoins doivent être présents sur chaque plaque :

- colonne 1 et 12 : témoin milieu
- colonne 2 et 11 : témoin milieu + bactéries

*Distribution du milieu MH*

100 µL dans chacun des puits des colonnes 1 et 12.
50 µL dans chacun des puits des colonnes 2 et 11.

*Distribution de la gamme de dilution de l'antibiotique à tester et de la molécule d'intérêt*

Cx1 : 25 µL dans les puits des colonnes 11 à 3, en commençant par la plus faible concentration.
ATB : 25 µL dans les puits des lignes H à A (colonnes 3 à 11), en commençant par la plus faible concentration.

*Distribution de la suspension bactérienne*

50 µL dans chacun des puits des colonnes 2 à 11.

**J2 : Lecture de la turbidité à 540 nm.**

Les différents types d'interactions observés par la technique de l'échiquier sont présentés dans les figures 2A, 2B, 2C et 2D :

La valeur FIC (Fractional Inhibitory Concentration Index) est déterminée par la formule suivante :

$$FIC = FIC_A + FIC_B = \frac{CMI_A \text{ en assoc}}{CMI_A \text{ seul}} + \frac{CMI_B \text{ en assoc}}{CMI_B \text{ seul}}$$

Lorsque FIC ≤ 0,5, il y a un effet synergique entre l'antibiotique A et l'antibiotique B.
Lorsque 0,5 < FIC ≤ 1, il y a un effet additif entre l'antibiotique A et l'antibiotique B.
Lorsque 1 < FIC ≤ 4, il y a un effet indifférent entre l'antibiotique A et l'antibiotique B.
Lorsque FIC > 4, il y a un effet antagoniste entre l'antibiotique A et l'antibiotique B.
Pour chaque souche et chaque combinaison antibiotique/Cx1, les expériences ont été répétées au minimum 3 fois.

**2. Résultats & Discussion**

Les résultats obtenus sont présentés sous forme de tableaux (Tableaux I à XII), présentant de façon plus détaillée, par couple ATB/Cx1 :

- les FIC index obtenus lors des différentes expériences ;
- les concentrations optimales pour la synergie ;
- les CMI des composés utilisés seuls ;
- les gammes évaluées et nature de l'interaction observée.

Les résultats ci-après montrent qu'aucun antagonisme entre le Cx1 et un antibiotique testé n'a été observé, quelque soit la souche et les associations testées.
Les résultats démontrent également que le traitement par du Cx1 en association avec le traitement par un antibiotique pour une pathologie impliquant une souche bactérienne présentant une résistance audit antibiotique permet :

- de conférer un certain niveau de sensibilité de la souche bactérienne vis-à-vis dudit antibiotique ;
- de diminuer la dose dudit antibiotique ainsi que la dose du Cx1 administrées.

Les résultats démontrent que le traitement par du Cx1 en association avec le traitement par un antibiotique pour une pathologie impliquant une souche bactérienne présentant une résistance à un antibiotique déterminé permet

de diminuer la dose d'antibiotique ainsi que la dose de Cx1 administrées.

Ces résultats sont également valables pour le traitement par du Cx1 en association avec le traitement par un antibiotique pour une pathologie impliquant une souche bactérienne présentant une résistance à au moins deux familles d'antibiotiques différentes. Tel est le cas des souches SaR3, AcR1, SaR4, PaR2 et PaR3 testées.

*Pseudomonas aeruginosa* est la souche, parmi toutes les souches analysées, sur laquelle le plus grand nombre d'associations synergiques, avec des antibiotiques très variés (β-lactamines, aminosides, fluoroquinolones...), a été observé.

Par ailleurs, les antibiotiques pour lesquels une synergie entre lesquels et le Cx1 a été observée agissent au niveau :

- de la paroi : fosfomycine (sachant que le mécanisme d'action spécifique de la fosfomycine se traduit par une synergie d'action quasi-constante avec les autres antibiotiques actifs sur la paroi bactérienne) mais aussi avec les associations pipéracilline-tazobactam & ticarcilline-acide clavulanique, et la ceftazidime ;
- de la synthèse des protéines : tigécycline, gentamicine, tobramycine, streptomycine, acide fusidique ;
- de la synthèse des acides nucléiques : rifampicine, ciprofloxacine

**2. Synergie de l'association du Cx1 avec des antibiotiques vis-à-vis de souches *d'Escherichia coli* résistantes ou non aux antibiotiques.**

**2.1. Tableau I : Synergie vis-à-vis de la souche *d'Escherichia coli* ATCC 25922 (phénotype sauvage) (n=4)**

| Associations | CMI initiales (mg/L) | Gammes testées (mg/L) | FIC index | CMI optimales (mg/L) | Différence CMI Cx1 | Différence CMI ATB | Conclusion |
|---|---|---|---|---|---|---|---|
| **Cx1/Amoxicilline** | 4/4 | 32/32 | 1-4 | nd | nd | Nd | Indifférence |
| **Cx1/Amoxicilline-Acide clavulanique** | 4/4 | 16/256 | 0,53-1 | 0,125/2 | ↓ x5 | ↓ x1 | Additivité |
| **Cx1/Pipéracilline** | 4/2 | 16/256 | 1-4 | nd | nd | nd | Indifférence |
| **Cx1/Céfotaxime** | 4/0,06 | 16/1 | 0,53-1 | 0,125/0,03 | ↓ x5 | ↓ x1 | Additivité |
| **Cx1/Ceftazidime** | 4/0,125 | 16/1 | 0,62-0,98 | 2/0,015 | ↓ x1 | ↓ x3 | Additivité |
| **Cx1/Imipénème** | 4/0,125 | 16/1 | 1-4 | nd | nd | nd | Indifférence |
| **Cx1/Ertapénème** | 4/0,015 | 16/1 | 0,625-1 | 1/0,07 | ↓ x2 | ↓ x1 | Additivité |
| **Cx1/Gentamicine** | 4/0,125 | 16/4 | 0,27-0,98 | 0,125/0,03 | ↓ x5 | ↓ x2 | Synergie |
| **Cx1/Amikacine** | 4/0,25 | 16/4 | 1-4 | nd | nd | nd | Indifférence |
| **Cx1/Tobramycine** | 4/0,25 | 16/4 | 0,365-1 | 0,5/0,06 | ↓ x3 | ↓ x2 | Synergie |
| **Cx1/Ciprofloxacine** | 2/0,015 | 16/1 | 1-4 | nd | nd | nd | Indifférence |
| **Cx1/Rifampicine** | 4/4 | 16/16 | 0,16-1 | 0,5/0,125 | ↓ x3 | ↓ x5 | Synergie |

**2.2. Tableau II : Synergie vis-à-vis d'une souche *d'Escherichia coli* productrice de pénicillinase sans résistance associée (ou EcR1) (n=4)**

| Associations | CMI initiales (mg/L) | Gammes testées (mg/L) | FIC index | Concentrations optimales (mg/L) | Différence CMI Cx1 | Différence CMI ATB | Conclusion |
|---|---|---|---|---|---|---|---|
| **Cx1/Amoxicilline** | 2/>256 | 32/256 | 1-4 | nd | nd | nd | Indifférence |
| **Cx1/Amoxicilline-Acide clavulanique** | 2/16 | 16/256 | 1-4 | nd | nd | nd | Indifférence |
| **Cx1/Pipéracilline** | 2/256 | 16/256 | 1-4 | nd | nd | nd | Indifférence |
| **Cx1/Céfotaxime** | 2/0,06 | 16/1 | 0,75-1 | 1/0,015 | ↓ x1 | ↓ x2 | Additivité |
| **Cx1/Ceftazidime** | 2/0,25 | 16/1 | 0,56-1 | 0,125/0,125 | ↓ x4 | ↓ x1 | Additivité |
| **Cx1/Imipénème** | 2/0,125 | 16/1 | 1-4 | nd | nd | nd | Indifférence |
| **Cx1/Ertapénème** | 2/0,015 | 16/1 | 0,75-1 | 0,5/0,07 | ↓ x2 | ↓ x1 | Additivité |
| **Cx1/Gentamicine** | 2/0,125 | 16/4 | 0,3-1 | 0,125/0,03 | ↓ x4 | ↓ x2 | Synergie |
| **Cx1/Amikacine** | 2/0,25 | 16/4 | 1-4 | nd | nd | nd | Indifférence |
| **Cx1/Tobramycine** | 2/0,25 | 16/4 | 1-4 | nd | nd | nd | Indifférence |
| **Cx1/Ciprofloxacine** | 2/0,015 | 16/1 | 1-4 | nd | nd | nd | Indifférence |
| **Cx1/Rifampicine** | 2/4 | 16/16 | 0,28-1 | 0,5/0,125 | ↓ x2 | ↓ x5 | Synergie |

**2.3. Tableau III : Synergie vis-à-vis d'une souche de *Escherichia coli* productrice de BLSE présentant une résistance associée aux aminosides (ou EcR3) (n=4)**

| Associations | CMI initiales (mg/L) | Gammes testées (mg/L) | FIC index | Concentrations optimales (mg/L) | Différence CMI Cx1 | Différence CMI ATB | Conclusion |
|---|---|---|---|---|---|---|---|
| **Cx1/Amoxicilline** | 2/>256 | 16/256 | 1-4 | nd | nd | nd | Indifférence |
| **Cx1/Amoxicilline-Acide clavulanique** | 2/32 | 16/256 | 0,56-1 | 0,125/16 | ↓ x4 | ↓ x1 | Additivité |
| **Cx1/Pipéracilline** | 2/128 | 16/256 | 1-4 | nd | nd | nd | Indifférence |
| **Cx1/Céfotaxime** | 2/128 | 16/256 | 0,375-1 | 0,25/32 | ↓ x3 | ↓ x2 | Synergie |
| **Cx1/Ceftazidime** | 2/1 | 16/16 | 0,56-1 | 0,125/0,5 | ↓ x4 | ↓ x1 | Additivité |
| **Cx1/Imipénème** | 2/0,125 | 16/1 | 1-4 | nd | nd | nd | Indifférence |
| **Cx1/Ertapénème** | 2/0,0015 | 16/1 | 1-4 | nd | nd | nd | Indifférence |
| **Cx1/Gentamicine** | 2/2 | 16/4 | 0,31-1 | 0,125/0,5 | ↓ x4 | ↓ x3 | Synergie |
| **Cx1/Amikacine** | 2/1 | 16/4 | 1-4 | nd | nd | nd | Indifférence |
| **Cx1/Tobramycine** | 2/4 | 16/4 | 1-4 | nd | nd | nd | Indifférence |
| **Cx1/Ciprofloxacine** | 2/0,06 | 16/1 | 0,56-1 | 0,125/0,03 | ↓ x4 | ↓ x1 | Additivité |
| **Cx1/Rifampicine** | 2/4 | 16/16 | 0,25-1 | 0,25/0,5 | ↓ x3 | ↓ x3 | Synergie |

**2.4. Tableau IV : Synergie vis-à-vis d'une souche de *Escherichia coli* hyperproductrice de céphalosporinase présentant une résistance associée aux aminosides, aux quinolones et à l'association triméthoprime-sulfaméthoxazole (ou EcR2) (n=4)**

| Associations | CMI initiales (mg/L) | Gammes testées (mg/L) | FIC index | Concentrations optimales (mg/L) | Différence CMI Cx1 | Différence CMI ATB | Conclusion |
|---|---|---|---|---|---|---|---|
| **Cx1/Amoxicilline** | 2/256 | 16/256 | 1-4 | nd | nd | nd | Indifférence |
| **Cx1/Amoxicilline-Acide clavulanique** | 2/256 | 32/256 | 0,56-1 | 0,125/128 | ↓ x4 | ↓ x1 | Additivité |
| **Cx1/Pipéracilline** | 2/32 | 16/256 | 0,56-1 | 0,125/16 | ↓ x4 | ↓ x1 | Additivité |
| **Cx1/Céfotaxime** | 2/4 | 16/32 | 1-4 | nd | nd | nd | Indifférence |
| **Cx1/Ceftazidime** | 2/8 | 16/32 | 1-4 | nd | nd | nd | Indifférence |
| **Cx1/Imipénème** | 2/0,125 | 16/1 | 1-4 | nd | nd | nd | Indifférence |
| **Cx1/Ertapénème** | 2/0,03 | 16/1 | 1-4 | nd | nd | nd | Indifférence |
| **Cx1/Gentamicine** | 2/2 | 16/4 | 0,31-1 | 0,125/0,5 | ↓ x4 | ↓ x2 | Synergie |
| **Cx1/Amikacine** | 2/0,5 | 16/4 | 1-4 | nd | nd | nd | Indifférence |
| **Cx1/Tobramycine** | 2/4 | 16/4 | 1-4 | nd | nd | nd | Indifférence |
| **Cx1/Ciprofloxacine** | 2/0,015 | 16/1 | 1-4 | nd | nd | nd | Indifférence |
| **Cx1/Rifampicine** | 2/2 | 16/16 | 0,31-1 | 0,25/0,5 | ↓ x3 | ↓ x2 | Synergie |

**3. Synergie de l'association du Cx1 avec des antibiotiques vis-à-vis de souches de *Staphylococcus aureus* résistantes ou non aux antibiotiques**

**3.1. Tableau V : Synergie vis-à-vis de la souche *Staphylococcus aureus* ATCC 29213 (phénotype sauvage) (n=4)**

| Associations | CMI initiales (mg/L) | Gammes testées (mg/L) | FIC index | Concentrations optimales (mg/L) | Différence CMI Cx1 | Différence CMI ATB | Conclusion |
|---|---|---|---|---|---|---|---|
| **Cx1/Pénicilline G** | 8/1 | 64/4 | 0,5-1 | 2/0,25 | ↓ x2 | ↓ x2 | Additivité |
| **Cx1/Imipénème** | 8/0,015 | 64/1 | 1-4 | nd | nd | nd | Indifférence |
| **Cx1/Erythromycine** | 8/0,5 | 64/4 | 1-4 | nd | nd | nd | Indifférence |
| **Cx1/Vancomycine** | 8/0,5 | 64/4 | 0,75-1 | 4/0,125 | ↓ x1 | ↓ x2 | Additivité |
| **Cx1/Lévofloxacine** | 8/0,125 | 64/4 | 1-4 | nd | nd | nd | Indifférence |
| **Cx1/Amikacine** | 8/1 | 64/4 | 0,53-1 | 4/0,03 | ↓ x1 | ↓ x5 | Additivité |
| **Cx1/Gentamicine** | 8/0,25 | 64/2 | 0,49-1 | 2/0,06 | ↓ x2 | ↓ x2 | Additivité |
| **Cx1/Streptomycine** | 8/4 | 64/32 | 0,375-1 | 1/1 | ↓ x3 | ↓ x2 | Synergie |
| **Cx1/Linézolide** | 8/2 | 64/8 | 1-4 | nd | nd | nd | Indifférence |
| **Cx1/Tigécycline** | 8/0,25 | 64/2 | 0,18-1 | 0,5/0,03 | ↓ x4 | ↓ x3 | Synergie |
| **Cx1/Acide fusidique** | 8/2 | 64/8 | 0,125-1 | 0,5/0,125 | ↓ x4 | ↓ x4 | Synergie |
| **Cx1/Fosfomycine** | 8/8 | 64/32 | 0,18-1 | 0,5/1 | ↓ x4 | ↓ x3 | Synergie |

**3.2. Tableau VI : Synergie vis-à-vis d'une souche de SARM sans résistance associée (ou SaR1) (n=4)**

| Associations | CMI (mg/L) | Gammes testées (mg/L) | FIC index | Concentrations optimales (mg/L) | Différence CMI Cx1 | Différence CMI ATB | Conclusion |
|---|---|---|---|---|---|---|---|
| **Cx1/Pénicilline G** | 8/0,5 | 64/4 | 0,5-1 | 2/0,125 | ↓ x2 | ↓ x2 | Additivité |
| **Cx1/Imipénème** | 8/0,25 | 64/4 | 0,625-1 | 0,5/0,06 | ↓ x4 | ↓ x2 | Additivité |
| **Cx1/Erythromycine** | 8/0,5 | 64/4 | 1-4 | nd | nd | nd | Indifférence |
| **Cx1/Vancomycine** | 8/0,25 | 64/4 | 1-4 | nd | nd | nd | Indifférence |
| **Cx1/Lévofloxacine** | 8/0,25 | 64/4 | 1-4 | nd | nd | nd | Indifférence |
| **Cx1/Gentamicine** | 8/0,25 | 64/2 | 0,49-1 | 2/0,06 | ↓ x2 | ↓ x2 | Additivité |
| **Cx1/Streptomycine** | 8/4 | 64/32 | 0,75-1 | 4/1 | ↓ x1 | ↓ x2 | Additivité |
| **Cx1/Linézolide** | 8/2 | 64/8 | 1-4 | nd | nd | nd | Indifférence |
| **Cx1/Tigécycline** | 8/0,25 | 64/2 | 0,18-1 | 0,5/0,03 | ↓ x4 | ↓ x3 | Synergie |
| **Cx1/Acide fusidique** | 8/0,5 | 64/8 | 0,245-1 | 1/0,06 | ↓ x3 | ↓ x3 | Synergie |
| **Cx1/Fosfomycine** | 8/2 | 64/32 | 0,31-1 | 0,5/0,5 | ↓ x4 | ↓ x2 | Synergie |

**3.3. Tableau VII : Synergie vis-à-vis d'une souche de SARM présentant une résistance aux aminosides et aux fluoroquinolones (SaR3) (n=4)**

| Associations | CMI (mg/L) | Gammes testées (mg/L) | FIC index | Concentrations optimales (mg/L) | Différence CMI Cx1 | Différence CMI ATB | Conclusion |
|---|---|---|---|---|---|---|---|
| **Cx1/Pénicilline G** | 8/0,5 | 64/4 | 0,375-1 | 2/0,06 | ↓ x2 | ↓ x3 | Synergie |
| **Cx1/Imipénème** | 8/0,25 | 64/4 | 0,56-1 | 2/0,06 | ↓ x2 | ↓ x2 | Additivité |
| **Cx1/Erythromycine** | 8/0,5 | 64/4 | 1-4 | nd | nd | nd | Indifférence |
| **Cx1/Vancomycine** | 8/0,25 | 64/4 | 1-4 | nd | nd | nd | Indifférence |
| **Cx1/Lévofloxacine** | 8/8 | 64/32 | 1-4 | nd | nd | nd | Indifférence |
| **Cx1/Gentamicine** | 8/0,125 | 64/2 | 0,74-1 | 4/0,03 | ↓ x1 | ↓ x2 | Additivité |
| **Cx1/Streptomycine** | 8/4 | 64/32 | 0,625-1 | 1/2 | ↓ x3 | ↓ x1 | Additivité |
| **Cx1/Linézolide** | 8/2 | 64/8 | 1-4 | nd | nd | nd | Indifférence |
| **Cx1/Tigécycline** | 8/0,25 | 64/2 | 0,245-1 | 1/0,03 | ↓ x3 | ↓ x3 | Synergie |
| **Cx1/Acide fusidique** | 8/0,25 | 64/8 | 0,365-1 | 1/0,06 | ↓ x3 | ↓ x2 | Synergie |
| **Cx1/Fosfomycine** | 8/16 | 64/32 | 0,18-1 | 0,5/2 | ↓ x4 | ↓ x3 | Synergie |

**3.4. Tableau VIII : Synergie vis-à-vis d'une souche de SARM présentant une résistance aux aminosides, aux fluoroquinolones, aux macrolides-lincosamides-synergistines et à l'ofloxacine (SaR4) (n=4)**

| Associations | CMI (mg/L) | Gammes testées (mg/L) | FIC index | Concentrations optimales (mg/L) | Différence CMI Cx1 | Différence CMI ATB | Conclusion |
|---|---|---|---|---|---|---|---|
| **Cx1/Pénicilline G** | 8/4 | 64/32 | 0,375-1 | 2/0,5 | ↓ x2 | ↓ x3 | Synergie |
| **Cx1/Imipénème** | 8/2 | 64/32 | 0,375-1 | 1/0,5<br>2/0,25 | ↓ x3<br>↓ x2 | ↓ x2<br>↓ x3 | Synergie |
| **Cx1/Erythromycine** | 8/>32 | 64/32 | 1-4 | nd | nd | nd | Indifférence |
| **Cx1/Vancomycine** | 8/0,25 | 64/4 | 1-4 | nd | nd | nd | Indifférence |
| **Cx1/Lévofloxacine** | 8/>32 | 64/32 | 1-4 | nd | nd | nd | Indifférence |
| **Cx1/Gentamicine** | 8/0,25 | 64/2 | 0,30-1 | 0,5/0,06 | ↓ x4 | ↓ x2 | Synergie |
| **Cx1/Streptomycine** | 8/4 | 64/32 | 0,625-1 | 4/0,5 | ↓ x1 | ↓ x3 | Additivité |
| **Cx1/Linézolide** | 8/2 | 64/8 | 0,56-1 | 0,5/1 | ↓ x4 | ↓ x1 | Additivité |
| **Cx1/Tigécycline** | 8/0,25 | 64/2 | 0,18-1 | 0,5/0,03 | ↓ x4 | ↓ x3 | Synergie |
| **Cx1/Acide fusidique** | 8/4 | 64/8 | 0,08-1 | 0,5/0,06 | ↓ x4 | ↓ x6 | Synergie |
| **Cx1/Fosfomycine** | 8/128 | 64/32 | 1-4 | nd | nd | nd | Indifférence |

**4. Synergie de l'association du Cx1 avec des antibiotiques vis-à-vis de souches de Pseudomonas aeruginosa résistantes ou non aux antibiotiques**

**4.1. Tableau IX : Synergie vis-à-vis de la souche *Pseudomonas aeruginosa* ATCC 27853 (phénotype sauvage) (n=4)**

| Associations | CMI (mg/L) | Gammes testées (mg/L) | FIC index | Concentrations optimales (mg/L) | Différence CMI Cx1 | Différence CMI ATB | Conclusion |
|---|---|---|---|---|---|---|---|
| **Cx1/Ticarcilline-Acide clavulanique** | 32/8 | 256/32 | 0,375-1 | 4/1 | ↓ x3 | ↓ x3 | Synergie |
| **Cx1/Pipéracilline-Tazobactam** | 32/16 | 256/128 | 0,31-1 | 2/4 | ↓ x4 | ↓ x2 | Synergie |
| **Cx1/Ceftazidime** | 32/4 | 256/64 | 0,375-1 | 4/1 | ↓ x3 | ↓ x2 | Synergie |
| **Cx1/Imipénème** | 32/2 | 256/16 | 0,5-4 | 8/0,5 | ↓ x2 | ↓ x2 | Additivité |
| **Cx1/Rifampicine** | 32/64 | 256/256 | 0,12-1 | 2/4 | ↓ x4 | ↓ x4 | Synergie |
| **Cx1/Colimycine** | 32/4 | 256/64 | 0,5-1 | nd | nd | nd | Additivité |
| **Cx1/Fosfomycine** | 32/16 | 256/256 | 0,375-1 | 4/4<br>2/8 | ↓ x3<br>↓ x4 | ↓ x2<br>↓ x1 | Synergie |
| **Cx1/Tobramycine** | 32/0,5 | 256/4 | 0,18-1 | 2/0,06 | ↓ x4 | ↓ x3 | Synergie |
| **Cx1/Amikacine** | 32/0,5 | 256/4 | 1-4 | nd | nd | nd | Indifférence |
| **Cx1/Ciprofloxacine** | 32/0,5 | 256/4 | 0,185-1 | 4/0,125 | ↓ x3 | ↓ x2 | Synergie |

**4.2. Tableau X : Synergie vis-à-vis d'une souche de *Pseudomonas aeruginosa* présentant une résistance aux β-lactamines, à l'association triméthoprime -sulfaméthoxazole et à la fosfomycine (ou PaR2) (n=4)**

| Associations | CMI (mg/L) | Gammes testées (mg/L) | FIC index | Concentrations optimales (mg/L) | Différence CMI Cx1 | Différence CMI ATB | Conclusion |
|---|---|---|---|---|---|---|---|
| **Cx1/Ticarcilline-Acide clavulanique** | 32/512 | 256/512 | 1-4 | nd | nd | nd | Indifférence |
| **Cx1/Pipéracilline-Tazobactam** | 32/512 | 256/512 | 0,25-1 | 4/64 | ↓ x3 | ↓ x3 | Synergie |
| **Cx1/Ceftazidime** | 32/8 | 256/64 | 1-4 | nd | nd | nd | Indifférence |
| **Cx1/Imipénème** | 32/2 | 256/16 | 0,5-4 | 8/1 | ↓ x2 | ↓ x1 | Additivité |
| **Cx1/Rifampicine** | 32/64 | 256/256 | 0,185-1 | 4/4<br>8/2 | ↓ x3<br>↓ x2 | ↓ x4<br>↓ x5 | Synergie |
| **Cx1/Colimycine** | 32/8 | 256/64 | 1-4 | nd | nd | nd | Indifférence |
| **Cx1/Fosfomycine** | 32/64 | 256/256 | 0,5-4 | 16/32 | ↓ x1 | ↓ x1 | Additivité |
| **Cx1/Tobramycine** | 32/1 | 256/4 | 0,31-1 | 2/0,25 | ↓ x4 | ↓ x2 | Synergie |
| **Cx1/Amikacine** | 32/1 | 256/4 | 1-4 | nd | nd | nd | Indifférence |
| **Cx1/Ciprofloxacine** | 32/1 | 256/4 | 0,5-4 | nd | nd | nd | Additivité |

**4.3. Tableau XI : Synergie vis-à-vis d'une souche de *Pseudomonas aeruginosa* présentant une résistance aux β-lactamines, (y compris les carbapénèmes), aux aminosides, à l'association triméthoprime-sulfaméthoxazole et à la ciprofloxacine (ou PaR3) (n = 4)**

| Associations | CMI (mg/L) | Gammes testées (mg/L) | FIC index | Concentrations optimales (mg/L) | Différence CMI Cx1 | Différence CMI ATB | Conclusion |
|---|---|---|---|---|---|---|---|
| **Cx1/Ticarcilline-Acide clavulanique** | 32/512 | 256/512 | 1-4 | nd | nd | nd | Indifférence |
| **Cx1/Pipéracilline-Tazobactam** | 32/512 | 256/512 | 1-4 | nd | nd | nd | Indifférence |
| **Cx1/Ceftazidime** | 32/16 | 256/64 | 0,31-1 | 2/4 | ↓ x4 | ↓ x2 | Synergie |
| **Cx1/Imipénème** | 32/32 | 256/256 | 0,5-1 | 8/8 | ↓ x2 | ↓ x2 | Additivité |
| **Cx1/Rifampicine** | 32/32 | 256/256 | 0,09-1 | 2/2 | ↓ x4 | ↓ x4 | Synergie |
| **Cx1/Colimycine** | 32/8 | 256/64 | 0,155-1 | 4/0,5 | ↓ x3 | ↓ x4 | Synergie |
| **Cx1/Fosfomycine** | 32/> 64 | 256/256 | 0,31-1 | 2/64 | ↓ x4 | >↓ x2 | Synergie |
| **Cx1/Tobramycine** | 32/64 | 256/256 | 0,185-1 | 2/8 | ↓ x4 | ↓ x3 | Synergie |
| **Cx1/Amikacine** | 32/1 | 256/4 | 1-4 | nd | nd | nd | Indifférence |
| **Cx1/Ciprofloxacine** | 32/0,5 | 256/4 | 0,5/4 | 8/0,125 | ↓ x2 | ↓ x2 | Additivité |

**4.4. Tableau XII : Synergie vis-à-vis d'une souche muqueuse de *Pseudomonas aeruginosa* présentant une résistance à la rifampicine et à l'association triméthoprime-sulfaméthoxazole (ou PaR5) (n=4)**

| Associations | CMI (mg/L) | Gammes testées (mg/L) | FIC index | Concentrations optimales (mg/L) | Différence CMI Cx1 | Différence CMI ATB | Conclusion |
|---|---|---|---|---|---|---|---|
| **Cx1/Ticarcilline-Acide clavulanique** | 32/32 | 256/512 | 1-4 | nd | nd | nd | Indifférence |
| **Cx1/Pipéracilline-Tazobactam** | 32/32 | 256/512 | 0,31-1 | 2/8 | ↓ x4 | ↓ x2 | Synergie |
| **Cx1/Ceftazidime** | 32/8 | 256/64 | 1-4 | nd | nd | nd | Indifférence |
| **Cx1/Imipénème** | 32/2 | 256/16 | 0,25-1 | 4/0,25 | ↓ x3 | ↓ x3 | Synergie |
| **Cx1/Rifampicine** | 32/16 | 256/256 | 0,185-1 | 2/2 | ↓ x4 | ↓ x3 | Synergie |
| **Cx1/Colimycine** | 32/16 | 256/64 | 0,187-1 | 2/2 | ↓ x4 | ↓ x3 | Synergie |
| **Cx1/Fosfomycine** | 32/16 | 256/256 | 0,375-1 | 8/2<br>4/4 | ↓ x2<br>↓ x3 | ↓ x3<br>↓ x2 | Synergie |
| **Cx1/Tobramycine** | 32/1 | 256/4 | 0,25-1 | 4/0,125 | ↓ x3 | ↓ x3 | Synergie |
| **Cx1/Amikacine** | 32/0,5 | 256/4 | 1-4 | nd | nd | nd | Indifférence |
| **Cx1/Ciprofloxacine** | 32/1 | 256/4 | 0,31-1 | 2/0,25 | ↓ x4 | ↓ x2 | Synergie |

**5. Identification et antibiogramme5.1 EcR1 : *Escherichia coli* productrice de pénicillinase sans résistance associée**

L'identification bactérienne et d'antibiogramme est effectuée par le système VITEK2 (bioMérieux) et par la technique de diffusion sur disques.

Tableau A1

| Antibiotiques | Diamètre | Dmin Dmax | Résultats | CMI mg/L | Résultats |
|---|---|---|---|---|---|
| Amoxicilline | 6 | 14-21 | Résistant | ≥ 32 | Résistant |
| Amox + ac.clavulanique | 20 | 14-21 | Intermédiaire | 4 | Sensible |
| Ticarcilline | 6 | 18-22 | Résistant | ≥ 128 | Résistant |
| Pipéracilline | 17 | 12-20 | Intermédiaire | ≤ 8 | Sensible |
| Piper + tazobactam | 25 | 14-21 | Sensible | ≤ 4 | Sensible |
| C1G | 17 | 12-18 | Intermédiaire | 4 | Sensible |
| Céfoxitine | 24 | 15-22 | Sensible | ≤ 4 | Sensible |
| Céfotaxime | 30 | 15-21 | Sensible | ≤ 1 | Sensible |
| Ceftazidime | | | | ≤ 1 | Sensible |
| Imipénème | | | | ≤ 1 | Sensible |
| Aztréonam | 28 | 17-23 | Sensible | | |
| Tobramycine | 19 | 14-16 | Sensible | ≤ 1 | Sensible |
| Gentamicine | 20 | 14-16 | Sensible | ≤ 1 | Sensible |
| Amikacine | 19 | 15-17 | Sensible | ≤ 2 | Sensible |
| Netilmicine | 24 | 17-19 | Sensible | ≤ 1 | Sensible |
| Minocycline | 20 | 17-19 | Sensible | | |
| Colistine | 15 | 15 | Sensible | | |
| Trimétoprime - Sulfamét. | 21 | 10-16 | Sensible | ≤ 20 | Sensible |
| Acide nalidixique | | | | ≤ 2 | Sensible |
| Norfloxacine | | | | ≤ 0,5 | Sensible |
| Ofloxacine | | | | ≤ 0,25 | Sensible |
| Péfloxacine | 26 | 16-22 | Sensible | | |
| Ciprofloxacine | 27 | 19-22 | Sensible | ≤ 0,25 | Sensible |
| Rifampicine | 16 | 14-19 | Intermédiaire | | |
| Fosfomycine | 24 | 14 | Sensible | | |
| Nitrofurantoïne | | | | ≤ 16 | Sensible |
| Céfépime | 27 | 15-21 | Sensible | | |

Tableau A2

| Antibiotiques | CMI mg/L (diamètre en mm) | Cc CA-SFM 2011 mg/L | Interprétation CA-SFM | Cc EUCAST 2011 | Interprétation EUCAST | Cc CLSI 2011 | Interprétation CLSI |
|---|---|---|---|---|---|---|---|
| Amoxicilline | ≥ 32 | 4-8 | **Résistant** | 8 | **Résistant** | 8-32 | **Résistant** |
| Amox + ac.clavulanique | 4 | 4-8 | Sensible | 8 | Sensible | 8-32 | Sensible |
| Ticarcilline | ≥ 128 | 8-16 | **Résistant** | 8-16 | **Résistant** | 16-128 | **Résistant** |
| Pipéracilline | ≤ 8 | 8-16 | Sensible | 8-16 | Sensible | 16-128 | Sensible |
| Piper + tazobactam | ≤ 4 | 8-16 | Sensible | 8-16 | Sensible | 16-128 | Sensible |
| Céfalotine | 4 | 8-32 | Sensible | 16 | Sensible | 8-32 | Sensible |
| Céfoxitine | ≤ 4 | 8-32 | Sensible | NA | - | 8-32 | Sensible |
| Céfotaxime | ≤ 1 | 1-2 | Sensible | 1-2 | Sensible | 1-4 | Sensible |
| Ceftazidime | ≤ 1 | 1-4 | Sensible | 1-4 | Sensible | 4-16 | Sensible |
| Céfépime | 27 | 24 | Sensible | 21-24 | Sensible | 14-18 | Sensible |
| Imipénème | ≤ 0.5 | 0.5-1 | Sensible | 2-8 | Sensible | 4-16 | Sensible |
| Aztréonam | 28 | 21-27 | Sensible | 24-27 | Sensible | 17-21 | Sensible |
| Tobramycine | ≤ 1 | 2-4 | Sensible | 2-4 | Sensible | 4-16 | Sensible |
| Gentamicine | ≤ 1 | 2-4 | Sensible | 2-4 | Sensible | 4-16 | Sensible |
| Amikacine | ≤ 2 | 8-16 | Sensible | 8-16 | Sensible | 16-64 | Sensible |
| Netilmicine | ≤ 1 | 2-4 | Sensible | 2-4 | Sensible | 8-32 | Sensible |
| Minocycline | 20 | 17-19 | Sensible | | - | 12-16 | Sensible |
| Colistine | 15 | 15 | Sensible | 17 | **Résistant** | nd | nd |
| Trimétoprime - Sulfamét. | ≤ 20 | 2-4 | Sensible | 2-4 | Sensible | 2-4 | Sensible |
| Acide nalidixique | ≤ 2 | 8-16 | Sensible | NA | - | 16-32 | Sensible |
| Norfloxacine | ≤ 0,5 | 0.5-1 | Sensible | 0.5-1 | Sensible | 4-16 | Sensible |
| Ofloxacine | ≤ 0,25 | 0.5-1 | Sensible | 0.5-1 | Sensible | 2-8 | Sensible |
| Ciprofloxacine | ≤ 0,25 | 0.5-1 | Sensible | - | - | 1-4 | Sensible |
| Rifampicine | 16 | 14-19 | **Intermédiaire** | - | - | nd | nd |
| Fosfomycine | 24 | 14 | Sensible | - | - | 12-16 | Sensible |
| Nitrofurantoïne | ≤ 16 | 64 | Sensible | 64 | Sensible | 32-128 | Sensible |

*Cc : Concentration critique

Conclusion expert juillet 2006 : Pénicillinase acquise

Les fluctuations relatives au type de résistance associée pour certaines souches entre les tableaux A1 et A2 sont dues à l'évolution des recommandations françaises (CA-SFM), européennes (EUCAST) et américaines (CLSI) en matière de catégorisation des souches bactériennes.

5.2 **EcR2 : *Escherichia coli* hyperproductrice de céphalosporinase présentant une résistance associée aux aminosides, aux quinolones et à l'association triméthoprime-sulfaméthoxazole**

L'identification bactérienne et d'antibiogramme est effectuée par le système VITEK1 (bioMérieux) et par la technique de diffusion sur disques

Tableau B1

| Antibiotiques | Diamètre | Dmin Dmax | Résultats | CMI mg/L |
|---|---|---|---|---|
| Amoxicilline | | | Résistant | ≥ 32 |
| Amox + ac.clavulanique | | | Résistant | ≥ 32 |
| Ticarcilline | | | Intermédiaire | 32 |
| C1G | | | Résistant | ≥ 64 |
| Céfoxitine | 11 | 15-22 | Résistant | |
| Céfotaxime | 24 | 15-21 | Intermédiaire | |
| Ceftazidime | 20 | 15-21 | Intermédiaire | |
| Imipénème | | | Sensible | ≤ 4 |
| Tobramycine | 10 | 16-18 | Résistant | |
| Gentamicine | 12 | 16-18 | Résistant | |
| Netilmicine | 21 | 19-21 | Sensible | ≤ 1 |
| Trimétoprime - Sulfamét. | | | Intermédiaire | 160 |
| Acide nalidixique | | | Résistant | ≥ 32 |
| Péfloxacine | | | Résistant | ≥ 8 |
| Nitrofurantoïne | | | Sensible | ≤ 25 |

Tableau B2

| Antibiotiques | CMI mg/L (diamètre en mm) | Cc*CA -SFM 2011 mg/L | Interprétation CA-SFM | Cc EUCAST 2011 | Interprétation EUCAST | Cc CLSI 2011 | Interprétatio n CLSI |
|---|---|---|---|---|---|---|---|
| Amoxicilline | ≥ 32 | 4-8 | **Résistant** | 8 | **Résistant** | 8-32 | **Résistant** |
| Amox + ac.clavulanique | ≥ 32 | 4-8 | **Résistant** | 8 | **Résistant** | 8-32 | **Résistant** |
| Ticarcilline | 32 | 8-16 | **Résistant** | 8-16 | **Résistant** | 16-128 | **Résistant** |
| Céfalotine | ≥ 64 | 8-32 | **Résistant** | 16 | **Résistant** | 8-32 | **Résistant** |
| Céfoxitine | 11 | 15-22 | **Résistant** | 19 | **Résistant** | 14-18 | **Résistant** |
| **Céfotaxime | 24 | 23-26 | **Intermédiaire** | 18-21 | **Résistant** | 22-26 | **Intermédiaire** |
| **Ceftazidime | 20 | 23-26 | **Résistant** | 19-22 | **Intermédiaire** | 17-21 | **Intermédiaire** |
| Imipénème | ≤ 0.5 | 0.5-1 | Sensible | 2-8 | Sensible | 4-16 | Sensible |
| Tobramycine | 10 | 16-18 | **Résistant** | 13-16 | **Résistant** | 12-15 | **Résistant** |
| **Gentamicine | 12 | 16-18 | **Résistant** | 14-17 | **Résistant** | 12-15 | **Résistant** |
| Netilmicine | ≤ 1 | 2-4 | Sensible | 2-4 | Sensible | 8-32 | Sensible |
| Trimétoprime - Sulfamét. | 160 | 2-4 | **Résistant** | 2-4 | **Résistant** | 8-16 | **Résistant** |
| Acide nalidixique | ≥ 32 | 8-16 | **Résistant** | - | - | 16-32 | **Résistant** |
| Péfloxacine | ≥ 8 | 1-4 | **Résistant** | - | - | - | - |
| Nitrofurantoïne | ≤ 25 | 64 | Sensible | 64 | Sensible | 32-128 | Sensible |

*Cc : Concentration critique

** Attention : la charge du disque est différente entre CA-SFM et EUCAST ; l'interprétation de l'EUCAST n'est donc pas applicable dans le présent cas. De plus, la technique de réalisation du test de diffusion en disque est différente dans les deux référentiels.

Les fluctuations relatives au type de résistance associée pour certaines souches entre les tableaux B1 et B2 sont dues à l'évolution des recommandations françaises (CA-SFM), européennes (EUCAST) et américaines (CLSI) en matière de catégorisation des souches bactériennes.

5.3 **EcR3 : *Escherichia coli* productrice de BLSE présentant une résistance associée aux aminosides, à la rifampicine et à l'association triméthoprime-sulfaméthoxazole**

L'identification bactérienne et d'antibiogramme est effectuée par le système VITEK2 (bioMérieux) et par la technique de diffusion sur disques

Tableau C1

| Antibiotiques | Diamètre | Dmin Dmax | Résultats |
|---|---|---|---|
| Amoxicilline | 6 | 14-21 | Résistant |
| Amox + ac.clavulanique | 16 | 14-21 | Intermédiaire |
| Ticarcilline | 6 | 18-22 | Résistant |
| Pipéracilline | 13 | 12-20 | Intermédiaire |
| Piper + tazobactam | | | Intermédiaire |
| C1G | 6 | 12-18 | Résistant |
| Céfoxitine | | | Intermédiaire |
| Céfotaxime | 19 | 15-21 | Intermédiaire |
| Imipénème | 27 | 17-22 | Sensible |
| Tobramycine | 6 | 14-16 | Résistant |
| Gentamicine | 12 | 14-16 | Résistant |
| Amikacine | 17 | 15-17 | Sensible |
| Netilmicine | 10 | 17-19 | Résistant |
| Minocycline | 21 | 17-19 | Sensible |
| Colistine | 16 | 15 | Sensible |
| Trimétoprime - Sulfamét. | 6 | 10-16 | Résistant |
| Péfloxacine | 23 | 16-22 | Sensible |
| Ciprofloxacine | 25 | 19-22 | Sensible |
| Rifampicine | 15 | 14-19 | Intermédiaire |
| Fosfomycine | 23 | 14 | Sensible |
| Céfépime | 24 | 15-21 | Intermédiaire |

Tableau C2

| Antibiotiques | diamètre en mm | Dc* CA-SFM 2011 | Interprétation CA-SFM | Dc EUCAST 2011 | Interprétation EUCAST | Dc CLSI 2011 | Interprétation CLSI |
|---|---|---|---|---|---|---|---|
| Amoxicilline | 6 | 16-19 | **Résistant** | 14 | **Résistant** | 13-17 | **Résistant** |
| Amox + ac.clavulanique | 16 | 16-21 | **Intermédiaire** | 17 | **Résistant** | 13-18 | **Intermédiaire** |
| Ticarcilline | 6 | 22-24 | **Résistant** | 22-23 | **Résistant** | 14-20 | **Résistant** |
| Pipéracilline | 13 | 16-20 | **Résistant** | 15-18 | **Résistant** | 17-21 | **Résistant** |
| **Piper + tazobactam | 18 | 17-21 | **Intermédiaire** | 15-18 | Sensible | 17-21 | **Intermédiaire** |
| Céfalotine | 6 | 12-18 | **Résistant** | - | - | 14-18 | **Résistant** |
| Céfoxitine | 6 | 15-22 | **Résistant** | 19 | **Résistant** | 14-18 | **Résistant** |
| **Céfotaxime | 19 | 23-26 | **Résistant** | 18-21 | **Intermédiaire** | 22-26 | **Résistant** |
| Céfépime | 24 | 24 | **Sensible** | 21-24 | Sensible | 14-18 | Sensible |
| Imipénème | 27 | 17-24 | Sensible | 15-21 | Sensible | 13-16 | Sensible |
| Tobramycine | 6 | 16-18 | **Résistant** | 13-16 | **Résistant** | 12-15 | **Résistant** |
| **Gentamicine | 12 | 16-18 | **Résistant** | 14-17 | **Résistant** | 12-15 | **Intermédiaire** |
| Amikacine | 17 | 15-17 | Sensible | 13-16 | Sensible | 14-17 | Sensible |
| **Netilmicine | 10 | 19-21 | **Résistant** | 12-15 | **Résistant** | 12-15 | **Résistant** |
| Minocycline | 21 | 17-19 | Sensible | - | - | 12-16 | Sensible |
| Colistine | 16 | 15 | Sensible | - | - | - | - |
| Trimétoprime - Sulfamét. | 6 | 13-16 | **Résistant** | 13-16 | **Résistant** | 10-16 | **Résistant** |
| Péfloxacine | 23 | 16-22 | Sensible | - | - | - | - |
| Ciprofloxacine | 25 | 22-25 | Sensible | - | - | 15-21 | Sensible |
| Rifampicine | 15 | 14-19 | **Intermédiaire** | - | - | - | - |
| Fosfomycine | 23 | 14 | Sensible | - | - | 12-16 | Sensible |

*Dc : Diamètre critique

** Attention : la charge du disque est différente entre CA-SFM et EUCAST ; l'interprétation de l'EUCAST n'est pas applicable dans le cas présent. De plus, la technique de réalisation du test de diffusion en disque est différente dans les deux référentiels.

Les fluctuations relatives au type de résistance associée pour certaines souches entre les tableaux C1 et C2 sont dues à l'évolution des recommandations françaises (CA-SFM), européennes (EUCAST) et américaines (CLSI) en matière de catégorisation des souches bactériennes.

### 5.4 SaR1 : *Staphylococcus aureus* Résistant à la Méticilline sans résistance associée

L'identification bactérienne et d'antibiogramme est effectuée par le système VITEK2 (bioMérieux)

Tableau D1

| Antibiotiques | Diamètre | Dmin Dmax | Résultats | CMI mg/L | Résultats |
|---|---|---|---|---|---|
| Pénicilline G | 28 | 9-29 | Résistant | ≥ 0,5 | Résistant |
| Oxacilline | | | | ≥ 8 | Résistant |
| Kanamycine | 20 | 15-17 | Sensible | ≤ 4 | Sensible |
| Tobramycine | 22 | 14-16 | Sensible | ≤ 1 | Sensible |
| Gentamicine | 23 | 14-16 | Sensible | ≤ 0,5 | Sensible |
| Chloramphénicol | 25 | 19-23 | Sensible | | |
| Minocycline | 27 | 17-19 | Sensible | | |
| Erythromycine | 25 | 17-22 | Sensible | ≤ 0,25 | Sensible |
| Lincomycine | 24 | 17-21 | Sensible | ≤ 1 | Sensible |
| Pristinamycine | 25 | 19-22 | Sensible | ≤ 0,5 | Sensible |
| Quinupristine-dalfopristine | | | | ≤ 0,25 | Sensible |
| Trimétoprime - Sulfamét. | 26 | 10-16 | Sensible | ≤ 10 | Sensible |
| Ofloxacine | 24 | 16-22 | Sensible | 1 | Sensible |
| Acide fusidique | 28 | 15-22 | Sensible | ≤ 0,5 | Sensible |
| Vancomycine | | | Sensible | ≤ 1 | Sensible |
| Teicoplanine | | | Sensible | ≤ 0,5 | Sensible |
| Rifampicine | 30 | 14-29 | Sensible | ≤ 0,5 | Sensible |
| Fosfomycine | 40 | 14 | Sensible | ≤ 8 | Sensible |
| Linézolide | 29 | 24-28 | Sensible | 2 | Sensible |
| Minocycline | | | | ≤ 0,5 | Sensible |
| Nitrofurantoïne | | | | ≤ 16 | Sensible |

Tableau D2

| Antibiotiques | CMI mg/L | Cc*CA-SFM 2011 mg/L | Interprétation CA-SFM | Cc EUCAST 2011 | Interprétation EUCAST | Cc CLSI 2011 | Interprétation CLSI |
|---|---|---|---|---|---|---|---|
| Pénicilline G | ≥ 0,5 | 0,12 | **Résistant** | 0,125 | **Résistant** | 0,12-0,25 | **Résistant** |
| Oxacilline | ≥ 8 | 2 | **Résistant** | 2 | **Résistant** | 2-4 | **Résistant** |
| Kanamycine | ≤ 4 | 8-16 | Sensible | 8-16 | Sensible | 16-64 | Sensible |
| Tobramycine | ≤ 1 | 1 | Sensible | 1 | Sensible | 4-16 | Sensible |
| Gentamicine | ≤ 0,5 | 1 | Sensible | 1 | Sensible | 4-16 | Sensible |
| Erythromycine | ≤ 0,25 | 1-2 | Sensible | 1-2 | Sensible | 0,5-8 | Sensible |
| Lincomycine | ≤ 1 | 2-8 | Sensible | - | - | - | - |
| Pristinamycine | ≤ 0,5 | 1-2 | Sensible | - | - | - | - |
| Quinupristine-dalfopristine | ≤ 0,25 | 1-2 | Sensible | 1-2 | Sensible | 1-4 | Sensible |
| Trimétoprime - Sulfamét. | ≤ 10 | 2-4 | Sensible | 2-4 | Sensible | 2-4 | Sensible |
| Ofloxacine | 1 | 1 | Sensible | 1 | Sensible | 1-4 | Sensible |
| Acide fusidique | ≤ 0,5 | 1 | Sensible | 1 | Sensible | | |
| Vancomycine | ≤ 1 | 2 | Sensible | 2 | Sensible | 4-32 | Sensible |
| Teicoplanine | ≤ 0,5 | 4 | Sensible | 2 | Sensible | 8-32 | Sensible |
| Rifampicine | ≤ 0,5 | 0,06-0,5 | Sensible | 0,064-0,5 | Sensible | 1-4 | Sensible |
| Fosfomycine | ≤ 8 | 32 | Sensible | 32 | Sensible | | - |
| Linézolide | 2 | 4 | Sensible | 4 | Sensible | 4-8 | Sensible |
| Minocycline | ≤ 0,5 | 0,5-1 | Sensible | 0,5-1 | Sensible | 4-16 | Sensible |
| Nitrofurantoïne | ≤ 16 | 64 | Sensible | 64 | Sensible | 32-128 | Sensible |

* Cc : Concentration critique

Février 2006 : Recherche du gène mecA par technique PCR : POSITIVE
Conclusion expert juillet 2006 : phénotype tout à fait typique : modification des PLP

Les fluctuations relatives au type de résistance associée pour certaines souches entre les tableaux D1 et D2 sont dues à l'évolution des recommandations françaises (CA-SFM), européennes (EUCAST) et américaines (CLSI) en matière de catégorisation des souches bactériennes.

5.5 **SaR3 : *Staphylococcus aureus* Résistant à la Méticilline présentant une résistance associée aux aminosides et aux fluoroquinolines**

L'identification bactérienne et d'antibiogramme est effectuée par le système VITEK2 (bioMérieux) et par la technique de diffusion sur disques

Tableau E1

| Antibiotiques | Diamètre | Dmin Dmax | Résultats |
|---|---|---|---|
| Pénicilline G Oxacilline | 26 | 9-29 | Résistant |
| Kanamycine | 6 | 15-17 | Résistant |
| Tobramycine | 6 | 14-16 | Résistant |
| Gentamicine | 20 | 14-16 | Sensible |
| Chloramphénicol | 6 | 19-23 | Résistant |
| Minocycline | 26 | 17-19 | Sensible |
| Erythromycine | 25 | 17-22 | Sensible |
| Lincomycine | 23 | 17-21 | Sensible |
| Pristinamycine | 23 | 19-22 | Sensible |
| Trimétoprime - Sulfamét. | 27 | 10-16 | Sensible |
| Ofloxacine | 6 | 16-22 | Résistant |
| Acide fusidique | 29 | 15-22 | Sensible |
| Vancomycine | | | Sensible |
| Teicoplanine | | | Sensible |
| Rifampicine | 31 | 14-29 | Sensible |
| Fosfomycine | 26 | 14 | Sensible |
| Linézolide | 26 | 24-28 | Sensible |

Tableau E2

| Antibiotiques | diamètre en mm | Dc* CA-SFM 2011 | Interprétation CA-SFM | Dc EUCAST 2011 | Interprétation EUCAST | Dc CLSI 2011 | Interprétation CLSI |
|---|---|---|---|---|---|---|---|
| Pénicilline G | 26 | | **Résistant** | 26 | **Résistant** | 28-29 | **Résistant** |
| Céfoxitine | 17 | 25-37 | **Résistant** | 22 | **Résistant** | 21-22 | **Résistant** |
| **Kanamycine | 6 | 15-17 | **Résistant** | 16-18 | **Résistant** | 13-18 | **Résistant** |
| Tobramycine | 6 | 20 | **Résistant** | 18 | **Résistant** | 12-15 | **Résistant** |
| **Gentamicine | 20 | 20 | Sensible | 18 | Sensible | 12-15 | Sensible |
| Erythromycine | 25 | 19-22 | Sensible | 18-21 | Sensible | 13-23 | Sensible |
| Lincomycine | 23 | 17-21 | Sensible | - | - | - | - |
| Pristinamycine | 23 | 19-22 | Sensible | - | - | - | - |
| Trimétoprime - Sulfamét. | 27 | 13-16 | Sensible | 14-17 | Sensible | 10-16 | Sensible |
| Ofloxacine | 6 | 22 | **Résistant** | 20 | **Résistant** | 14-18 | **Résistant** |
| Acide fusidique | 29 | 24 | Sensible | 24 | Sensible | - | - |
| Vancomycine | 28 | 17 | Sensible | - | - | - | - |
| Teicoplanine | 27 | 17 | Sensible | - | - | 10-14 | Sensible |
| Rifampicine | 31 | 24-29 | Sensible | 23-26 | Sensible | 16-20 | Sensible |
| Fosfomycine | 26 | 14 | Sensible | - | - | - | - |
| **Linézolide | 26 | 24 | Sensible | 19 | Sensible | 20-21 | Sensible |
| Minocycline | 26 | 21-23 | Sensible | 20-23 | Sensible | 14-19 | Sensible |

* Dc : Diamètre critique

** Attention : la charge du disque est différente entre CA-SFM et EUCAST ; l'interpétation d'EUCAST n'est dons pas applicable dans le cas présent. De plus, la technique de réalisation du test de diffusion en disque est différente dans les deux référentiels.

Les fluctuations relatives au type de résistance associée pour certaines souches entre les tableaux E1 et E2 sont dues à l'évolution des recommandations françaises (CA-SFM), européennes (EUCAST) et américaines (CLSI) en matière de catégorisation des souches bactériennes.

5.6 **SaR4 : *Staphylococcus aureus* Résistant à la Méticilline présentant une résistance associée aux aminosides, aux fluoroquinolones, aux macrolides-lincosamines-synergistines et à l'ofloxacine**

L'identification bactérienne et d'antibiogramme est effectuée par le système VITEK2 (bioMérieux) et par la technique de diffusion sur disques

Tableau F1

| Antibiotiques | Diamètre | Dmin Dmax | Résultats | CMI mg/L |
|---|---|---|---|---|
| Pénicilline G | | | Résistant | |
| Oxacilline | | | Résistant | ≥ 8 |
| Kanamycine | 7 | 15-17 | Résistant | |
| Tobramycine | 6 | 20-20 | Résistant | |
| Gentamicine | 21 | 20-20 | Sensible | |
| Chloramphénicol | | | Sensible | 8 |
| Tétracycline | | | Sensible | ≤ 1 |
| Minocycline | | | Sensible | ≤ 4 |
| Erythromycine | | | Résistant | ≥ 8 |
| Lincomycine | | | Résistant | ≥ 16 |
| Pristinamycine | | | Sensible | ≤ 2 |
| Trimétoprime - Sulfamét. | | | Sensible | ≤ 10 |
| Ofloxacine | | | Résistant | ≥ 8 |
| Nitrofurantoïne | | | Sensible | ≤ 25 |
| Acide fusidique | | | Sensible | ≤ 1 |
| Vancomycine | | | Sensible | 1 |
| Teicoplanine | | | Sensible | ≤ 4 |
| Rifampicine | | | Sensible | ≤ 1 |
| Fosfomycine | | | Résistant | ≥ 64 |

Tableau F2

| Antibiotiques | CMI mg/L (diamètre en mm) | Cc*CA-SFM 2011 mg/L | Interprétation CA-SFM | Cc EUCAST 2011 | Interprétation EUCAST | Cc CLSI 2011 | Interprétation CLSI |
|---|---|---|---|---|---|---|---|
| Pénicilline G | | | **Résistant** | | **Résistant** | 0,12-0,25 | **Résistant** |
| Oxacilline | ≥ 8 | 2 | **Résistant** | 2 | **Résistant** | 2-4 | **Résistant** |
| **Kanamycine | 7 | 15-17 | **Résistant** | 16-18 | **Résistant** | 13-18 | **Résistant** |
| Tobramycine | 6 | 20 | **Résistant** | 18 | **Résistant** | 12-15 | **Résistant** |
| **Gentamicine | 21 | 20 | Sensible | 18 | Sensible | 12-15 | Sensible |
| Tétracycline | ≤ 1 | 1-2 | Sensible | 1-2 | Sensible | 4-16 | Sensible |
| Minocycline | ≤ 0,5 | 0,5-1 | Sensible | 0,5-1 | Sensible | 4-16 | Sensible |
| Erythromycine | ≥ 8 | 1-2 | **Résistant** | 1-2 | **Résistant** | 0,5-8 | **Résistant** |
| Lincomycine | ≥ 16 | 2-8 | **Résistant** | - | - | - | - |
| Pristinamycine | ≤ 2 | 1-2 | Sensible | - | - | - | - |
| Trimétoprim - Sulfamét. | ≤ 10 | 2-4 | Sensible | 2-4 | Sensible | 2-4 | Sensible |
| Ofloxacine | ≥ 8 | 1 | **Résistant** | 1 | **Résistant** | 1-4 | **Résistant** |
| Nitrofurantoïne | ≤ 25 | 64 | Sensible | 64 | Sensible | 32-128 | Sensible |
| Acide fusidique | ≤ 1 | 1 | Sensible | 1 | Sensible | - | - |
| Vancomycine | 1 | 2 | Sensible | 2 | Sensible | 4-32 | Sensible |
| Teicoplanine | ≤ 4 | 4 | Sensible | 2 | Sensible | 8-32 | Sensible |
| Rifampicine | ≤ 1 | 0,06-0,5 | Sensible | 0,064-0,5 | Sensible | 1-4 | Sensible |
| Fosfomycine | ≥ 64 | 32 | **Résistant** | 32 | **Résistant** | - | - |

*Cc : Concentration critique

** Attention : la charge du disque est différente entre CA-SFM et EUCAST ; l'interprétation de l'EUCAST n'est donc pas applicable dans le cas présent. De plus, la technique de réalisation du test de diffusion en disque est différente dans les deux référentiels.

Les fluctuations relatives au type de résistance associée pour certaines souches entre les tableaux F1 et F2 sont dues à l'évolution des recommandations françaises (CA-SFM), européennes (EUCAST) et américaines (CLSI) en matière de catégorisation des souches bactériennes.

**5.7 PaR2 : *Pseudomonas aeruginosa* présentant une résistance associée aux β-lactamines, à l'association triméthoprime-sulfaméthoxazole et à la fosfomycine**

L'identification bactérienne et d'antibiogramme est effectuée par le système VITEK2 (bioMérieux) et par la technique de diffusion sur disques

Tableau G1

| Antibiotiques | Diamètre | Dmin-Dmax | Résultats |
|---|---|---|---|
| Ticar + ac. clavulanique | 10 | 18-22 | Résistants |
| Ticarcilline | 10 | 18-22 | Résistant |
| Pipéracilline | 22 | 12-18 | Sensible |
| Piper + tazobactam | 22 | 14-19 | Sensible |
| Ceftazidime | 23 | 15-21 | Sensible |
| Aztréonam | 16 | 17-23 | Résistant |
| Imipénème | 27 | 17-22 | Sensible |
| Tobramycine | 24 | 14-16 | Sensible |
| Gentamicine | 21 | 14-16 | Sensible |
| Amikacine | 22 | 15-17 | Sensible |
| Nétilmicine | 19 | 17-19 | Sensible |
| Minocycline | 6 | 17-19 | Résistant |
| Colistine | 20 | 15-15 | Sensible |
| Trimétoprime - Sulfamét. | 6 | 10-16 | Résistant |
| Péfloxacine | 8 | 16-22 | Résistant |
| Rifampicine | 25 | 19-22 | Sensible |
| Fosfomycine | 13 | 14-19 | Résistant |
| Ciprofloxacine | 18 | 14-14 | Sensible |
| Céfépime | 19 | 15-21 | Intermédiaire |

Tableau G2

| Antibiotiques | diamètre en mm | Dc* CA-SFM 2011 | Interprétation CA-SFM | Dc EUCAST 2011 | Interprétation EUCAST | Dc CLSI 2011 | Interprétation CLSI |
|---|---|---|---|---|---|---|---|
| Ticar + ac. clavulanique | 10 | 22 | **Résistant** | 17 | **Résistant** | 14-15 | **Résistant** |
| Ticarcilline | 10 | 22 | **Résistant** | 17 | **Résistant** | 14-15 | **Résistant** |
| **Pipéracilline | 22 | 18 | Sensible | 19 | Sensible | 17-18 | Sensible |
| **Piper+ tazobactam | 22 | 19 | Sensible | 19 | Sensible | 17-18 | Sensible |
| **Ceftazidime | 23 | 19 | Sensible | 16 | Sensible | 14-18 | Sensible |
| Céfépime | 19 | 19 | Sensible | 18 | Sensible | 14-18 | Sensible |
| Aztréonam | 16 | 19-27 | **Résistant** | 16-50 | **Résistant** | 15-22 | **Intermédiaire** |
| Imipénème | 27 | 17-22 | Sensible | 17-20 | Sensible | 13-16 | Sensible |
| Tobramycine | 24 | 16 | Sensible | 16 | Sensible | 12-15 | Sensible |
| **Gentamicine | 21 | 16 | Sensible | 15 | Sensible | 12-15 | Sensible |
| Amikacine | 22 | 15-17 | Sensible | 15-18 | Sensible | 14-17 | Sensible |
| **Nétilmicine | 19 | 19 | Sensible | 12 | Sensible | 12-15 | Sensible |
| Minocycline | 6 | - | **Résistant** | - | - | - | - |
| Colistine | 20 | - | Sensible | - | - | 10-11 | Sensible |
| Trimétoprime-Sulfamét. | 6 | - | **Résistant** | 16 | **Résistant** | - | - |
| Ciprofloxacine | 25 | 22-25 | Sensible | 22-25 | Sensible | 15-21 | Sensible |
| Rifampicine | 25 | 14-19 | Sensible | - | - | - | - |
| Fosfomycine | 13 | 14 | **Résistant** | - | - | - | - |

*Dc : Diamètre critique

** Attention : la charge du disque est différente entre CA-SFM et EUCAST; l'interprétation de l'EUCAST n'est donc pas applicable dans le cas présent. De plus, la technique de réalisation du test de diffusion en disque est différente dans les deux référentiels.

Les fluctuations relatives au type de résistance associée pour certaines souches entre les tableaux G1 et G2 sont dues à l'évolution des recommandations françaises (CA-SFM), européennes (EUCAST) et américaines (CLSI) en matière de catégorisation des souches bactériennes.

5.8 **PaR3 : *Pseudomonas aeruginosa* présentant une résistance associée aux β-lactamines, aux aminosides (y compris les carbapénémes), à l'association triméthoprime-sulfaméthoxazole et à la ciprofloxacine**

L'identification bactérienne et d'antibiogramme est effectuée par le système VITEK2 (bioMérieux)

Tableau H1

| **Antibiotiques** | **CMI** | **Résultats** |
|---|---|---|
| Ticar + ac. clavulanique | ≥ 128 | Résistant |
| Ticarcilline | ≥ 128 | Résistant |
| Pipéracilline | ≥ 128 | Résistant |
| Piper + tazobactam | ≥ 128 | Résistant |
| Ceftazidime | 4 | Sensible |
| Aztréonam | 16 | Sensible |
| Imipénème | ≥ 16 | Résistant |
| Méropénème | ≥ 16 | Résistant |
| Tobramycine | ≥ 16 | Résistant |
| Gentamicine | ≥ 16 | Résistant |
| Amikacine | 4 | Sensible |
| Minocycline | 4 | Résistant |
| Colistine | ≤ 0,5 | Sensible |
| Trimétoprime - Sulfamét. | ≥ 320 | Résistant |
| Péfloxacine | 4 | Intermédiaire |
| Ciprofloxacine | 1 | Sensible |
| Céfépime | 16 | Intermédiaire |

Tableau H2

| Antibiotiques | CMI mg/L | Cc*CA-SFM 2011 mg/L | Interprétation CA-SFM | Cc EUCAST 2011 | Interprétation EUCAST | Cc CLSI 2011 | Interprétation CLSI |
|---|---|---|---|---|---|---|---|
| Ticar + ac. clavulanique | ≥ 128 | 16 | **Résistant** | 16 | **Résistant** | 64-128 | **Résistant** |
| Ticarcilline | ≥ 128 | 16 | **Résistant** | 16 | **Résistant** | 64-128 | **Résistant** |
| Pipéracilline | ≥ 128 | 16 | **Résistant** | 16 | **Résistant** | 64-128 | **Résistant** |
| Piper + tazobactam | ≥ 128 | 16 | **Résistant** | 16 | **Résistant** | 64-128 | **Résistant** |
| Ceftazidime | 4 | 8 | Sensible | 8 | Sensible | 8-32 | Sensible |
| Céfépime | 16 | 8 | **Résistant** | 8 | **Résistant** | 8-32 | **Intermédiaire** |
| Aztréonam | 16 | 1-16 | **Résistant** | 1-16 | **Résistant** | 8-32 | **Intermédiaire** |
| Imipénème | ≥ 16 | 4-8 | **Résistant** | 4-8 | **Résistant** | 4-16 | **Résistant** |
| Méropénème | ≥ 16 | 2-8 | **Résistant** | 2-8 | **Résistant** | 4-16 | **Résistant** |
| Tobramycine | ≥ 16 | 4 | **Résistant** | 4 | **Résistant** | 4-16 | **Résistant** |
| Gentamicine | ≥ 16 | 4 | **Résistant** | 4 | **Résistant** | 4-16 | **Résistant** |
| Amikacine | 4 | 8-16 | Sensible | 8-16 | Sensible | 16-64 | Sensible |
| Minocycline | 4 | - | **Résistant** | - | - | - | - |
| Colistine | ≤ 0,5 | 2-4 | Sensible | 4 | Sensible | 2-8 | Sensible |
| Trimétoprime - Sulfamét. | ≥ 320 | - | **Résistant** | 4 | **Résistant** | - | - |
| Ciprofloxacine | 1 | 0,5-1 | **Intermédiaire** | 0,5-1 | **Intermédiaire** | 1-4 | Sensible |

*Cc : concentration critique

Les fluctuations relatives au type de résistance associée pour certaines souches entre les tableaux H1 et H2 sont dues à l'évolution des recommandations françaises (CA-SFM), européennes (EUCAST) et américaines (CLSI) en matière de catégorisation des souches bactériennes.

5.9 **PaR5 : *Pseudomonas aeruginosa* présentant une résistance associée à la rifampicine et à l'association triméthoprime-sulfaméthoxazole**

L'identification bactérienne et d'antibiogramme est effectuée par le système VITEK2 (bioMérieux) et par la technique de diffusion sur disques

Tableau 11

| Antibiotiques | Diamètre | Dmin-Dmax | Résultats |
|---|---|---|---|
| Ticar + ac. clavulanique | 40 | 18-22 | Sensible |
| Ticarcilline | 32 | 18-22 | Sensible |
| Pipéracilline | 33 | 12-18 | Sensible |
| Piper + tazobactam | 40 | 14-19 | Sensible |
| Ceftazidime | 32 | 15-21 | Sensible |
| Aztréonam | 33 | 17-23 | Sensible |
| Imipénème | 25 | 17-22 | Sensible |
| Méropénème | 34 | 15-20 | Sensible |
| Tobramycine | 28 | 14-16 | Sensible |
| Gentamicine | 25 | 14-16 | Sensible |
| Amikacine | 27 | 15-17 | Sensible |
| Nétilmicine | 28 | 17-19 | Sensible |
| Minocycline | 11 | 17-19 | Résistant |
| Colistine | 23 | 15 | Sensible |
| Trimétoprime - Sulfamét. | 6 | 10-16 | Résistant |
| Norofloxacine | 17 | 22-25 | Résistant |
| Ofloxacine | 11 | 22-25 | Résistant |
| Ciprofloxacine | 30 | 19-22 | Sensible |
| Rifampicine | 13 | 14-19 | Résistant |
| Fosfomycine | 30 | 14 | Sensible |
| Céfépime | 32 | 15-21 | Sensible |

Tableau I2

| Antibiotiques | diamètre en mm | Dc* CA-SFM 2011 | Interprétation CA-SFM | Dc EUCAST 2011 | Interprétation EUCAST | Dc CLSI 2011 | Interprétation CLSI |
|---|---|---|---|---|---|---|---|
| Ticar+ac. clavulanique | 40 | 22 | Sensible | 17 | Sensible | 14-15 | Sensible |
| Ticarcilline | 32 | 22 | Sensible | 17 | Sensible | 14-15 | Sensible |
| **Pipéracilline | 33 | 18 | Sensible | 19 | Sensible | 17-18 | Sensible |
| **Piper+ tazobactam | 40 | 19 | Sensible | 19 | Sensible | 17-18 | Sensible |
| **Ceftazidime | 32 | 19 | Sensible | 16 | Sensible | 14-18 | Sensible |
| Céfépime | 32 | 19 | Sensible | 18 | Sensible | 14-18 | Sensible |
| Aztréonam | 33 | 19-27 | Sensible | 16-50 | **Intermédiaire** | 15-22 | Sensible |
| Imipénème | 25 | 17-22 | Sensible | 17-20 | Sensible | 13-16 | Sensible |
| Méropénème | 34 | 15-22 | Sensible | 18-24 | Sensible | 13-16 | Sensible |
| Tobramycine | 28 | 16 | Sensible | 16 | Sensible | 12-15 | Sensible |
| **Gentamicine | 25 | 16 | Sensible | 15 | Sensible | 12-15 | Sensible |
| Amikacine | 27 | 15-17 | Sensible | 15-18 | Sensible | 14-17 | Sensible |
| **Nétilmicine | 28 | 19 | Sensible | 12 | Sensible | 12-15 | Sensible |
| Minocycline | 11 | - | **Résistant** | - | - | - | - |
| Colistine | 23 | - | Sensible | - | - | 10-11 | Sensible |
| Trimétoprime - Sulfamét. | 6 | - | **Résistant** | 16 | **Résistant** | - | - |
| Ciprofloxacine | 30 | 22-25 | Sensible | 22-25 | Sensible | 15-21 | Sensible |
| Rifampicine | 13 | 14-19 | **Résistant** | - | - | - | - |
| Fosfomycine | 30 | 14 | Sensible | - | - | - | - |

*Dc : diamètre critique

** Attention : la charge du disque est différente entre CA-SFM et EUCAST; l'interprétation de l'EUCAST n'est donc pas applicable dans le cas présent. De plus la technique de réalisation du test de diffusion en disque est différente dans les deux référentiels.

Les fluctuations relatives au type de résistance associée pour certaines souches entre les tableaux I1 et I2 sont dues à l'évolution des recommandations françaises (CA-SFM), européennes (EUCAST) et américaines (CLSI) en matière de catégorisation des souches bactériennes

### 6. Synergie de l'association du Cx1 avec des antiseptiques vis-à-vis des souches bactériennes de phénotype sauvage

6.1. Synergie vis-à-vis de la souche d'*E. coli* ATCC 25922 (phénotype sauvage)

| Associations | CMI initiales (mg/L) | Gammes testées (mg/L) | FIC index | CMI optimales (mg/L) | Différence CMI Cx1 | Différence CMI ATS | Conclusion |
|---|---|---|---|---|---|---|---|
| **Cx1/Hexamidine** | 4/8 | 64/32 | >1 | nd | nd | nd | Indifférence |
| **Cx1/Chlorhexidine** | 4/<1 | 64/4 | >1 | nd | nd | nd | Indifférence |

6.2 Synergie vis-à-vis de la souche *S. aureus* ATCC 29213 (phénotype sauvage)

| Associations | CMI initiales (mg/L) | Gammes testées (mg/L) | FIC index | CMI optimales (mg/L) | Différence CMI Cx1 | Différence CMI ATS | Conclusion |
|---|---|---|---|---|---|---|---|
| **Cx1/Hexamidine** | 8/<1 | 64/4 | >1 | nd | nd | nd | Indifférence |
| **Cx1/Chlorhexidine** | 8/<1 | 64/4 | >1 | nd | nd | nd | Indifférence |

6.3. Synergie vis-à-vis de la souche *P. aeruginosa* ATCC 27853 (phénotype sauvage)

| Associations | CMI initiales (mg/L) | Gammes testées (mg/L) | FIC index | CMI optimales (mg/L) | Différence CMI Cx1 | Différence CMI ATS | Conclusion |
|---|---|---|---|---|---|---|---|
| **Cx1/Hexamidine** | 32/32 | 64/256 | 3 | nd | nd | nd | Indifférence |
| **Cx1/Chlorhexidine** | 32/4 | 64/32 | 1.5 | nd | nd | nd | Indifférence |

**Revendications**

**1.** Produit comprenant au moins un antibiotique donné et un calixarène représenté par la formule I suivante :

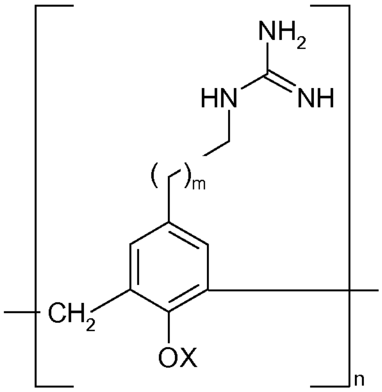

Formule I

,

dans laquelle :

(i) n = un entier de 4 à 16,
(ii) m= un entier de 1 à 10,
(iii) X est choisi parmi :

- un hydrogène,
- un groupement alkyl, le nombre de carbones étant de 1 à 20, notamment de 1 à 10,
- un halogène choisi parmi Cl, Br, I, ou
- un groupement amphiphile choisi parmi un groupe anionique, tels que les carboxylates $-RCO_2^-$, les sulfates $-RSO_4^-$, les sulfonates $-RSO_3^-$, un groupe cationique, tel que $RNH_3^+$, dans lesquels R est un groupement alkyl, le nombre de carbones étant de 1 à 20, notamment de 1 à 10,

pour son utilisation comme médicament.

**2.** Produit pour utilisation selon la revendication 1, dans lequel le calixarène correspond au calixarène représenté par la formule II suivante :

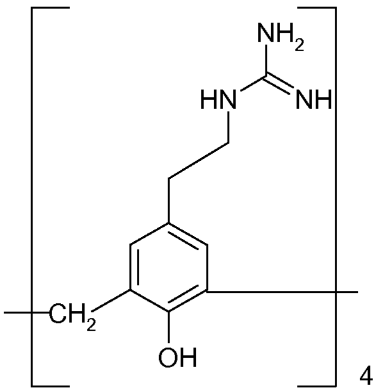

Formule II

**3.** Produit pour utilisation selon la revendication 1 ou 2, dans lequel ledit antibiotique donné est choisi dans le groupe constitué des β- lactamines, des aminosides, des fluoroquinolones, de la fosfomycine, de la colimycine, de la rifampicine, de la tigécycline, ou de l'acide fusidique.

**4.** Produit pour utilisation selon la revendication 3, dans lequel ledit antibiotique donné est choisi parmi l'imipénème, la pipéracilline-tazobactam, la pénicilline G, le céfotaxime, la ceftazidime, la tobramycine, la gentamicine, la ciprofloxacine, la rifampicine, la fosfomycine, la colimycine, la streptomycine, la ticarcilline-acide clavulanique, la tigécycline ou l'acide fusidique.

**5.** Produit pour utilisation selon l'une des revendications 1 à 4, comme médicament dans le traitement des pathologies impliquant une souche bactérienne présentant une résistance à au moins un antibiotique déterminé.

**6.** Produit pour utilisation selon la revendication 5, dans le traitement des pathologies impliquant une souche bactérienne résistante appartenant à une espèce choisie parmi *Escherichia coli, Pseudomonas aeruginosa,* et *Staphylococcus aureus.*

**7.** Produit pour utilisation selon la revendication 6, dans le traitement des pathologies impliquant une souche bactérienne résistante choisie parmi :

- une souche de *Staphylococcus aureus* de phénotype sauvage,
- une souche de *Staphylococcus aureus* Résistant à la Méticilline (SARM) sans résistance associée,
- une souche de SARM présentant une résistance aux aminosides et aux fluoroquinolones,
- une souche de SARM présentant une résistance aux aminosides, aux fluoroquinolones, aux macrolides-lincosamides-synergistines et à l'ofloxacine,
- une souche d'*Escherichia coli* de phénotype sauvage,
- une souche d'*Escherichia coli* productrice de BLSE (β-Lactamase à Spectre Etendu) présentant une résistance associée aux aminosides, à la rifampicine et à l'association triméthoprime-sulfaméthoxazo le,
- une souche d'*Escherichia coli* productrice de pénicillinase sans résistance associée,
- une souche d'*Escherichia coli* hyperproductrice de céphalosporinase présentant une résistance associée aux aminosides, aux quinolones et à l'association triméthoprime-sulfaméthoxazole,
- une souche de *Pseudomonas aeruginosa* de phénotype sauvage,
- une souche de *Pseudomonas aeruginosa* présentant une résistance aux β-lactamines, à l'association triméthoprime-sulfaméthoxazole et à la fosfomycine,
- une souche de *Pseudomonas aeruginosa* présentant une résistance aux β-lactamines (y compris les carbapénémes), aux aminosides, à l'association triméthoprime-sulfaméthoxazole et à la ciprofloxacine,
- une souche muqueuse de *Pseudomonas aeruginosa* présentant une résistance à la rifampicine et à l'association triméthoprime-sulfaméthoxazole.

**8.** Produit pour utilisation selon l'une des revendications 5 à 7, dans le traitement des pathologies appartenant au groupe constitué des infections nosocomiales et/ou communautaires, telles que les infections abdominales, les infections digestives, les infections urinaires, les infections respiratoires, les infections neuro-méningées, les infections de la sphère oro-pharyngée, les infections génitales, les endocardites, les infections de la peau et des tissus mous, les infections ostéoarticulaires, les infections oculaires, les septicémies ou bactériémies.

**9.** Composition pharmaceutique comprenant comme substance active au moins un produit selon l'une quelconque des revendications 1 à 8 en association avec un véhicule pharmaceutiquement acceptable.

**10.** Produit contenant :

- un antibiotique donné choisi dans le groupe constitué des β-lactamines, des aminosides, des fluoroquinolones, de la fosfomycine, de la colimycine, de la rifampicine,de la tigécycline, ou de l'acide fusidique, et
- un calixarène représenté par la formule I suivante :

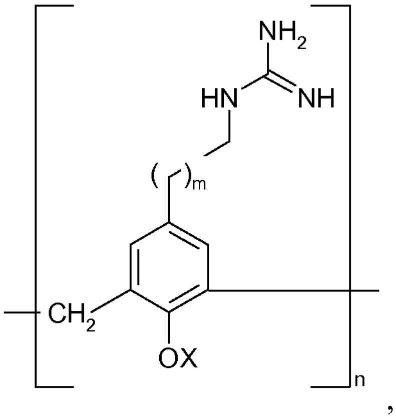

Formule I

dans laquelle :

(i) n = un entier de 4 à 16,
(ii) m= un entier de 1 à 10,
(iii) X est choisi parmi :

- un hydrogène,
- un groupement alkyl, le nombre de carbones étant de 1 à 20, notamment de 1 à 10,
- un halogène choisi parmi Cl, Br, I, ou
- un groupement amphiphile choisi parmi un groupe anionique, tels que les carboxylates $-RCO_2^-$, les sulfates $-RSO_4^-$, les sulfonates $-RSO_3^-$, un groupe cationique, tel que $RNH_3^+$, dans lesquels R est un groupement alkyl, le nombre de carbones étant de 1 à 20, notamment de 1 à 10,

comme produit de combinaison, pour une utilisation simultanée, séparée ou étalée dans le temps en thérapie de pathologies impliquant au moins une souche bactérienne présentant une résistance à au moins un antibiotique déterminé, telles que les infections abdominales, les infections digestives, les infections urinaires, les infections respiratoires, les infections neuro-méningées, les infections de la sphère oro-pharyngée, les infections génitales, les endocardites, les infections de la peau et des tissus mous, les infections ostéoarticulaires, les infections aoculaires, les septicémies ou bactériémies.

**11.** Produit de combinaison pour utilisation selon la revendication 10, dans lequel le calixarène correspond au calixarène représenté par la formule II suivante :

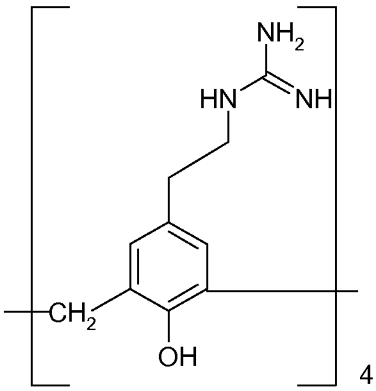

Formule II

**12.** Produit de combinaison pour utilisation selon la revendication 10 ou 11, dans lequel ledit antibiotique donné est choisi parmi l'imipénème, la pipéracilline-tazobactam, la pénicilline G, le céfotaxime, la ceftazidime, la tobramycine, la gentamicine, la ciprofloxacine, la rifampicine, la fosfomycine, la colimycine, la streptomycine, la ticarcilline-acide clavulanique, la tigécycline ou l'acide fusidique.

**13.** Produit de combinaison pour utilisation selon l'une quelconque des revendications 10 à 12, simultanée, séparée

ou étalée dans le temps en thérapie de pathologies impliquant au moins une souche bactérienne résistante appartenant à une espèce choisie parmi *Escherichia coli, Pseudomonas aeruginosa,* et *Staphylococcus aureus.*

**14.** Produit de combinaison pour utilisation selon la revendication 13, simultanée, séparée ou étalée dans le temps en thérapie de pathologies impliquant au moins une souche bactérienne résistante choisie parmi :

- une souche de *Staphylococcus aureus* de phénotype sauvage,
- une souche de *Staphylococcus aureus* Résistant à la Méticilline (SARM) sans résistance associée,
- une souche de SARM présentant une résistance aux aminosides et aux fluoroquinolones,
- une souche de SARM présentant une résistance aux aminosides, aux fluoroquinolones, aux macrolides-lincosamides-synergistines et à l'ofloxacine
- une souche *d'Escherichia coli* de phénotype sauvage,
- une souche *d'Escherichia coli* productrice de BLSE (β-Lactamase à Spectre Etendu) présentant une résistance associée aux aminosides, à la rifampicine et à l'association triméthoprime-sulfaméthoxazo le,
- une souche *d'Escherichia coli* productrice de pénicillinase sans résistance associée,
- une souche *d'Escherichia coli* hyperproductrice de céphalosporinase présentant une résistance associée aux aminosides, aux quinolones et à l'association triméthoprime-sulfaméthoxazole,
- une souche de *Pseudomonas aeruginosa* de phénotype sauvage,
- une souche de *Pseudomonas aeruginosa* présentant une résistance aux β-lactamines, à l'association triméthoprime-sulfaméthoxazole et à la fosfomycine,
- une souche de *Pseudomonas aeruginosa* présentant une résistance aux β-lactamines (y compris les carbapénémes), aux aminosides, à l'association triméthoprime-sulfaméthoxazole et à la ciprofloxacine,
- une souche muqueuse de *Pseudomonas aeruginosa* présentant une résistance à la rifampicine et à l'association triméthoprime-sulfaméthoxazole.

**Patentansprüche**

**1.** Produkt, das mindestens ein gegebenes Antibiotikum und ein Calixaren umfasst, welches durch die folgende Formel I dargestellt wird:

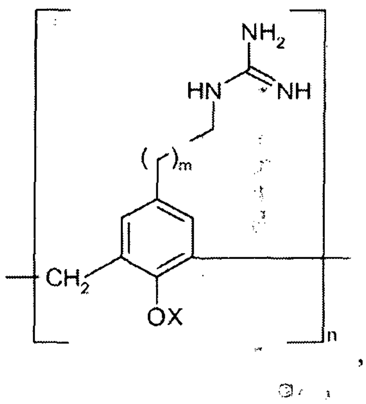

Formel I

wobei:

(i) n = eine ganze Zahl von 4 bis 16,
(ii) m = eine ganze Zahl von 1 bis 10,
(iii) X ausgewählt ist aus:

- Wasserstoff,
- einer Alkylgruppe, wobei die Anzahl der Kohlenstoffe 1 bis 20 beträgt, insbesondere 1-10,
- einem Halogen, ausgewählt aus Cl, Br, I, oder
- einer amphiphilen Gruppe, ausgewählt aus einer anionischen Gruppe, wie etwa Carboxylaten $-RCO_2^-$, Sulfaten $-RSO_4^-$, Sulfonaten $-RSO_3^-$, einer kationischen Gruppe, wie etwa $RNH_3^+$, wobei R eine Alkylgruppe ist, wobei die Anzahl von Kohlenstoffen 1 bis 20 beträgt, insbesondere 1 bis 10,

zur Verwendung als Arzneimittel.

2. Produkt zur Verwendung nach Anspruch 1, wobei das Calixaren dem durch die folgende Formel II dargestellten Calixaren entspricht:

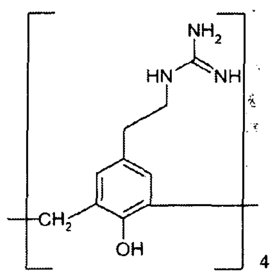

Formel II

3. Produkt zur Verwendung nach Anspruch 1 oder 2, wobei das gegebene Antibiotikum ausgewählt ist aus der Gruppe bestehend aus β-Lactam-Antibiotika, Aminoglykosiden, Fluorchinolonen, Fosfomycin, Colimycin, Rifampicin, Tigecyclin oder Fusidinsäure.

4. Produkt zur Verwendung nach Anspruch 3, wobei das gegebene Antibiotikum ausgewählt ist aus Imipenem, Piperacillin-Tazobactam, Penicillin G, Cefotaxim, Ceftazidim, Tobramycin, Gentamycin, Ciprofloxacin, Rifampicin, Fosfomycin, Colimycin, Streptomycin, Ticarcillin-Clavulansäure,Tigecyclin oder Fusidinsäure.

5. Produkt zur Verwendung nach einem der Ansprüche 1 bis 4 als Arzneimittel bei der Behandlung von Erkrankungen, die mit einem Bakterienstamm einhergehen; der eine Resistenz gegen mindestens ein gegebenes Antibiotikum aufweist.

6. Produkt zur Verwendung nach Anspruch 5 bei der Behandlung von Erkrankungen, die mit einem resistenten Bakterienstamm einhergehen, der einer Spezies, ausgewählt unter *Escherichia coli, Pseudomonas aeruginosa* und *Staphylococcus aureus* angehört.

7. Produkt zur Verwendung nach Anspruch 6, bei der Behandlung von Erkrankungen, die mit einem resistenten Bakterienstamm einhergehen, der ausgewählt ist aus:

- einem *Staphylococcus aureus* Stamm mit wildtypischem Phänotyp,
- einem gegen Methicillin (MRSA) resistenten *Staphyloccccus aureus* Stamm ohne assoziierte Resistenz,
- einem MRSA-Stamm, der eine Resistenz gegen Aminoglykoside und Fluorchinolone aufweist,
- einem MRSA-Stamm, der eine Resistenz gegen Aminoglykoside, Fluorchinolone, Makrolide-Lincosamide-Synergystine und gegen Ofloxacin aufweist,
- einem *Escherichia coli* Stamm mit wildtypischem Phänotyp,
- einem ESBL (β-Lactamase mit breitem Wirkungsspektrum)- produzierenden *Escherichia coli* Stamm mit einer Resistenz gegen Aminoglykoside, Rifampicin und der Verbindung Trimethoprim-Sulfamethoxazol,
- einem Penicillinase produzierenden *Escherichia coli* Stamm ohne assoziierte Resistenz,
- einem Cephalosporinase hochproduzierenden *Escherichia coli* Stamm mit einer Resistenz gegen Aminoglykoside, Chinolone und der Verbindung Trimethoprim-Sulfamethoxazol,
- einem *Pseudomonas aeruginosa* Stamm mit wildtypischem Phänotyp,
- einem *Pseudomonas aeruginosa* Stamm mit einer Resistenz gegen β-Lactam-Antibiotika, der Verbindung Trimethoprim-Sulfamethoxazol und Fosfomycin,
- einem Pseudomonas aeruginosa Stamm mit einer Resistenz gegen β-Lactam-Antibiotika (einschließlich Carbapeneme), Aminoglykoside, der Verbindung Trimethoprim-Sulfamethoxazol und Ciprofloxacin,
- einem *Pseudomonas aeruginosa* Schleimhaut-Stamm mit einer Resistenz gegen Rifampicin und der Verbindung Trimethoprim-Sulfamethoxazol.

8. Produkt zur Verwendung nach einem der Ansprüche 5 bis 7 bei der Behandlung von Erkrankungen der Gruppe bestehend aus nosokomialen und/oder Gemeinschaftsinfektionen, wie etwa abdominalen Infektionen, gastrointestinalen Infektionen, Harnwegsinfektionen, Atemwegsinfektionen, Neuro-Meningitis Infektionen, Infektionen des Mund-Rachen-Bereichs, Genitalinfektionen, Endokarditis, Infektionen der Haut und der Weichgewebe, Knochen-

und Gelenksinfektionen, Augeninfektionen, Sepsen oder Bakteriämien.

**9.** Pharmazeutische Zusammensetzung, die als Wirkstoff mindestens ein Produkt nach einem der Ansprüche 1 bis 8 in Verbindung mit einem pharmazeutisch verträglichen Träger umfasst.

**10.** Produkt, das Folgendes enthält:

- ein gegebenes Antibiotikum ausgewählt aus der Gruppe bestehend aus β-Lactam-Antibiotika, Aminoglykosiden, Fluorchinolonen, Fosfomycin, Colimycin, Rifampicin, Tigecyclin oder Fusidinsäure, und
- ein Calixaren, das durch die folgenden Formel I dargestellt wird:

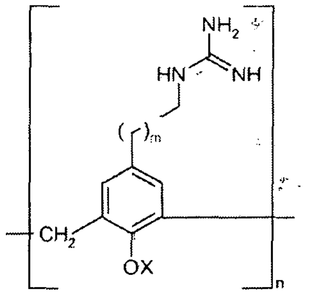

Formel I

wobei:

(i) n = eine ganze Zahl von 4 bis 16,
(ii) m = eine ganze Zahl von 1 bis 10,
(iii) X ausgewählt ist aus:

- Wasserstoff,
- einer Alkylgruppe, wobei die Anzahl der Kohlenstoffe 1 bis 20 beträgt, insbesondere 1-10 beträgt,
- einem Halogen, ausgewählt aus Cl, Br, I, oder
- einer amphiphilen Gruppe, ausgewählt aus einer anionischen Gruppe, wie etwa Carboxylaten $-RCO_2^-$, Sulfaten $-RSO_4^-$, Sulfonaten $-RSO_3^-$, einer kationischen Gruppe, wie etwa $RNH_3^+$, wobei R eine Alkylgruppe ist, wobei die Anzahl von Kohlenstoffen 1 bis 20 beträgt, insbesondere 1 bis 10;

als Kombinationspräparat zur gleichzeitigen, getrennten oder zeitlich verzögerten Anwendung bei der Therapie von Erkrankungen, die mit mindestens einem Bakterienstamm mit einer Resistenz gegen mindestens ein bestimmtes Antibiotikum einhergehen, wie etwa abdominalen Infektionen, gastrointestinalen Infektionen, Harnwegsinfektionen, Atemwegsinfektionen, Neuro-Meningitis Infektionen, Infektionen des Mund-Rachen-Bereichs, Genitalinfektionen, Endokarditis, Infektionen der Haut und der Weichgewebe, Knochen- und Gelenksinfektionen, Augeninfektionen, Sepsen oder Bakteriämien.

**11.** Kombinationspräparat zur Verwendung nach Anspruch 10, wobei das Calixaren dem durch die folgende Formel II dargestellten Calixaren entspricht:

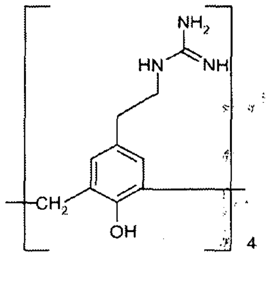

Formel II

**12.** Kombinationsprodukt zur Verwendung nach Anspruch 10 oder 11, wobei das gegebene Antibiotikum ausgewählt ist aus Imipenem, Piperacillin-Tazobactam, Penicillin G, Cefotaxim, Ceftazidim, Tobramycin, Gentamycin, Ciprofloxacin, Rifampicin, Fosfomycin, Colimycin, Streptomycin, Ticarcillin-Clavulansäure, Tigecyclin oder Fusidinsäure.

**13.** Kombinationsprodukt zur Verwendung nach einem der Ansprüche 10 bis 12, gleichzeitig, getrennt oder zeitlich verzögert, bei der Therapie von Erkrankungen, die mit mindestens einem resistenten Bakterienstamm einhergehen, der zu einer Spezies gehört, die ausgewählt ist aus *Escherichia coli, Pseudomonas aeruginosa* und *Staphylococcus aureus.*

**14.** Kombinationsprodukt zur Verwendung nach Anspruch 13, gleichzeitig, getrennt oder zeitlich verzögert, bei der Therapie von Erkrankungen, die mit mindestens einem resistenten Bakterienstamm einhergehen, der ausgewählt ist aus:

- einem *Staphylococcus aureus* Stamm mit wildtypischem Phänotyp,
- einem gegen Methicillin (MRSA) resistenten *Staphylococcus aureus* Stamm ohne assoziierte Resistenz, f
- einem MRSA-Stamm, der eine Resistenz gegen Aminoglykoside und Fluorchinolone aufweist,
- einem MRSA-Stamm, der eine Resistenz gegen Aminoglykoside, Fluorchinolone, Makrolide-Lincosamide-Synergystine und gegen Ofloxacin aufweist,
- einem *Escherichia coli* Stamm mit wildtypischem Phänotyp,
- einem ESBL (β-Lactamase mit breitem Wirkungsspektrum) -produzierenden Stamm von *Escherichia coli* mit einer Resistenz gegen Aminoglykoside, Rifampicin und der Verbindung Trimethoprim-Sulfamethoxazol,
- einem Penicillinase produzierenden *Escherichia coli* Stamm ohne assoziierte Resistenz,
- einem Cephalosporinase hochproduzierenden Stamm von *Escherichia coli* mit einer Resistenz gegen Aminoglykoside, Chinolone und der Verbindung Trimethoprim-Sulfamethoxazol,
- einem *Pseudomonas aeruginosa* Stamm mit wildtypischem Phänotyp,
- einem Stamm von *Pseudomonas aeruginosa* mit einer Resistenz gegen β-Lactam-Antibiotika, der Verbindung Trimethoprim-Sulfamethoxazol und Fosfomycin,
- einem *Pseudomonas aeruginosa* Stamm mit einer Resistenz gegen β-Lactam-Antibiotika (einschließlich Carbapeneme), Aminoglykoside, der Verbindung Trimethoprim-Sulfamethoxazol und Ciprofloxacin,
- einem *Pseudomonas aeruginosa* Schleimhaut-Stamm mit einer Resistenz gegen Rifampicin und der Verbindung Trimethoprim-Sulfamethoxazol.

## Claims

**1.** Product comprising at least one given antibiotic and a calixarene represented by Formula I below:

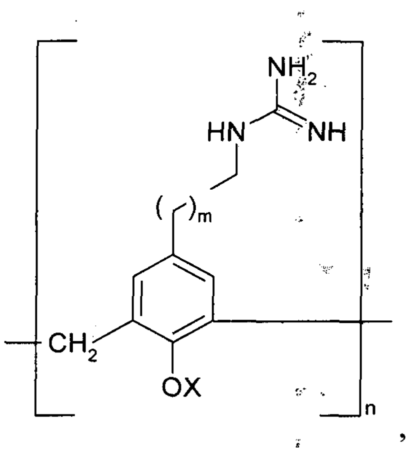

Formula I

in which:

(i) n = an integer from 4 to 16,
(ii) m = an integer from 1 to 10,
(iii) X is chosen from:

- a hydrogen,

- an alkyl group, the number of carbons being from 1 to 20, in particular from 1 to 10,
- a halogen chosen from Cl, Br, I, or
- an amphiphilic group chosen from an anionic group, such as the carboxylates $-RCO_2^-$, the sulphates $-RSO_4^-$, the sulphonates $-RSO_3^-$, a cationic group, such as $RNH_3^+$, in which R is an alkyl group, the number of carbons being from 1 to 20, in particular from 1 to 10,

for its use as medicament.

2. Product for use according to claim 1, in which the calixarene corresponds to the calixarene represented by Formula II below:

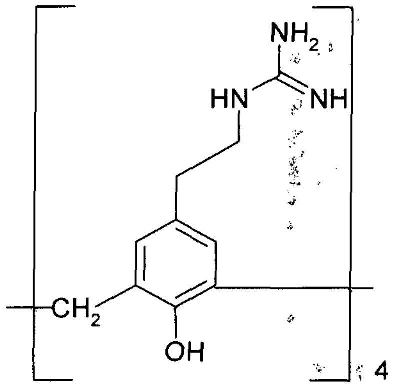

Formula II

3. Product for use according to claim 1 or 2, in which said given antibiotic is chosen from the group constituted by the β-lactams, the aminoglycosides, fluoroquinolones, fosfomycin, colimycin, rifampicin, tigecycline, or fusidic acid.

4. Product for use according to claim 3, in which said given antibiotic is chosen from imipenem, piperacillin-tazobactam, penicillin G, cefotaxime, ceftazidime, tobramycin, gentamicin, ciprofloxacin, rifampicin, fosfomycin, colimycin, streptomycin, ticarcillin-clavulanic acid, tigecycline or fusidic acid.

5. Product for use according to one of claims 1 to 4, as a medicament in the treatment of the pathologies involving a bacterial strain having a resistance to at least one defined antibiotic.

6. Product for use according to claim 5, in the treatment of the pathologies involving a resistant bacterial strain belonging to a species chosen from *Escherichia coli, Pseudomonas aeruginosa*, and *Staphylococcus aureus.*

7. Product for use according to claim 6, in the treatment of the pathologies involving a resistant bacterial strain chosen from:

- a wild-type strain of *Staphylococcus aureus*,
- a Methicillin-Resistant *Staphylococcus aureus* strain (MRSA) without associated resistance,
- an MRSA strain having a resistance to the aminoglycosides and fluoroquinolones,
- an MRSA strain having a resistance to the aminoglycosides, fluoroquinolones, macrolides-lincosamides-synergistins and ofloxacin,
- a wild-type strain of *Escherichia coli,*
- an ESBL (Extended-Spectrum β-Lactamase)-producing strain of *Escherichia coli* having an associated resistance to the aminoglycosides, rifampicin and the trimethoprime-sulphamethoxazole combination,
- a penicillinase-producing strain of *Escherichia coli* without associated resistance,
- a cephalosporinase-hyperproducing strain of *Escherichia coli,* having an associated resistance to the aminoglycosides, quinolones and the trimethoprime-sulphamethoxazole combination,
- a wild-type strain of *Pseudomonas aeruginosa*,
- a *Pseudomonas aeruginosa* strain having a resistance to the β-lactams, the trimethoprime-sulphamethoxazole combination and fosfomycin,
- a *Pseudomonas aeruginosa* strain having a resistance to the β-lactams (including the carbapenems), the aminoglycosides, the trimethoprime-sulphamethoxazole combination and ciprofloxacin,

- a mucoid strain of *Pseudomonas aeruginosa* having a resistance to rifampicin and the trimethoprime-sulphamethoxazole combination.

**8.** Product for use according to one of claims 5 to 7, in the treatment of the pathologies belonging to the group constituted by the nosocomial and/or community-acquired infections, such as abdominal infections, digestive infections, urinary infections, respiratory infections, neuro-meningeal infections, infections of the oro-pharyngeal sphere, genital infections, endocarditis, infections of the skin and of the soft tissues, osteo-articular infections, ocular infections, septicaemia or bacteraemia.

**9.** Pharmaceutical composition comprising as active substance at least one product according to any one of claims 1 to 8 in combination with a pharmaceutically acceptable vehicle.

**10.** Product containing:

- a given antibiotic chosen from the group constituted by the β-lactams, the aminoglycosides, fluoroquinolones, fosfomycine, colimycin, rifampicin, tigecycline, or fusidic acid, and
- a calixarene represented by Formula I below:

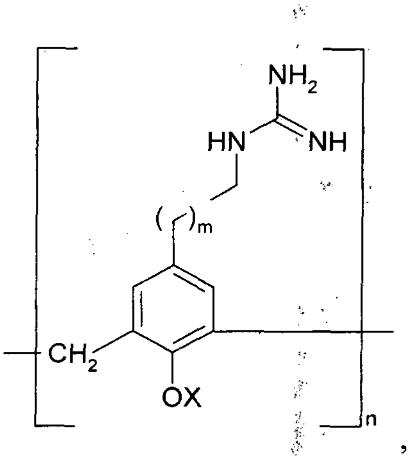

Formula I

,

in which:

(i) n = an integer from 4 to 16,
(ii) m = an integer from 1 to 10,
(iii) X is chosen from:

- a hydrogen,
- an alkyl group, the number of carbons being from 1 to 20, in particular from 1 to 10,
- a halogen chosen from Cl, Br, I, or
- an amphiphilic group chosen from an anionic group, such as the carboxylates $-RCO_2^-$, the sulphates $-RSO_4^-$, the sulphonates $-RSO_3^-$, a cationic group, such as $RNH_3^+$, in which R is an alkyl group, the number of carbons being from 1 to 20, in particular from 1 to 10,

as a combination product, for simultaneous or separate or spread over time use for the treatment of pathologies involving at least one bacterial strain having a resistance to at least one defined antibiotic, such as abdominal infections, digestive infections, urinary infections, respiratory infections, neuro-meningeal infections, infections of the oro-pharyngeal sphere, genital infections, endocarditis, infections of the skin and soft tissues, osteoarticular infections, ocular infections, septicaemia or bacteriaemia.

**11.** Combination product for use according to claim 10, in which the calixarene corresponds to the calixarene represented by Formula II below:

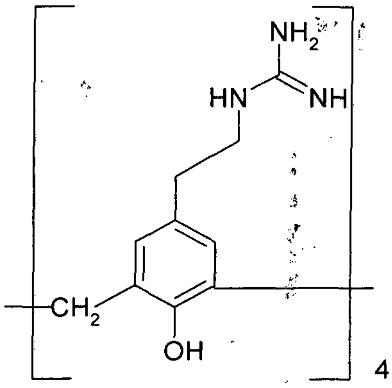

Formula II

**12.** Combination product for use according to claim 10 or 11, in which said given antibiotic is chosen from imipenem, piperacillin-tazobactam, penicillin G, cefotaxime, ceftazidime, tobramycin, gentamicin, ciprofloxacin, rifampicin, fosfomycin, colimycin, streptomycin, ticarcilline-clavulanic acid, tigecycline or fusidic acid.

**13.** Combination product for use according to any one of claims 10 to 12, for simultaneous or separate or spread over time use for the treatment of pathologies involving at least one resistant bacterial strain belonging to a species chosen from *Escherichia coli, Pseudomonas aeruginosa*, and *Staphylococcus aureus.*

**14.** Combination product for use according to claim 13, simultaneous, separate or spread over time in the treatment of the pathologies involving a resistant bacterial strain chosen from:

- a wild-type strain of *Staphylococcus aureus*,
- a strain of meticillin-resistant *Staphylococcus aureus* (MRSA) without associated resistance,
- a strain of MRSA having a resistance to the aminoglycosides and fluoroquinolones,
- a strain of MRSA having a resistance to the aminoglycosides, fluoroquinolones, macrolides-lincosamides-synergystins and ofloxacin,
- =a wild-type strain of *Escherichia coli,*
- an ESBL (extended spectrum β-Lactamase)-producing strain of *Escherichia coli,* having an associated resistance to the aminoglycosides, rifampicin and the trimethoprime-sulphamethoxazole combination,
- a penicillinase-producing strain of *Escherichia coli* without associated resistance,
- a cephalosporinase-hyperproducing strain of *Escherichia coli* having an associated resistance to the aminoglycosides, quinolones and the trimethoprime-sulphamethoxazole combination,
- a wild-type strain of *Pseudomonas aeruginosa*,
- a strain of *Pseudomonas aeruginosa* having a resistance to the β-lactams, the trimethoprime-sulphamethoxazole combination and fosfomycin,
- a strain of *Pseudomonas aeruginosa* having a resistance to the β-lactams (including the carbapenems), aminoglycosides, the trimethoprime-sulphamethoxazole combination and ciprofloxacin,
- a mucoid strain of *Pseudomonas aeruginosa* having a resistance to rifampicin and the trimethoprime-sulphamethoxazole combination.

Figure 1

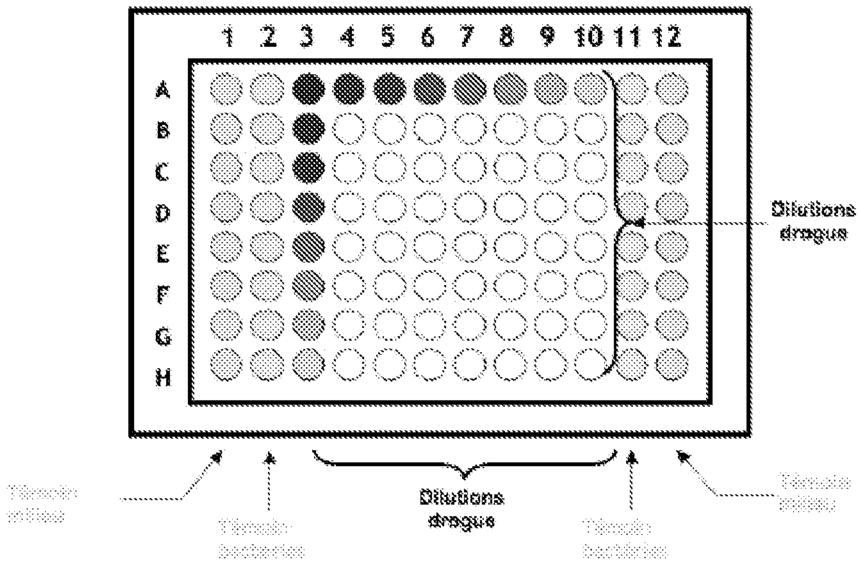


Figure 2A

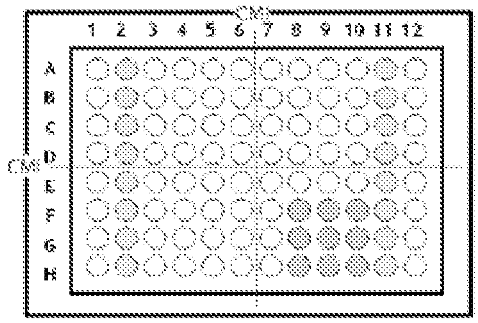


Figure 2B

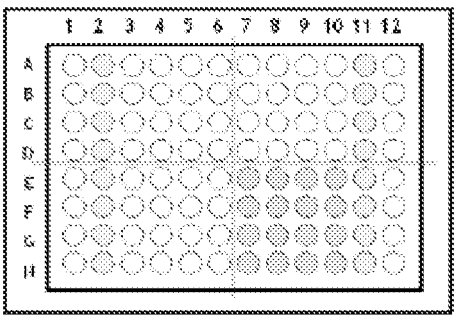

Figure 2C

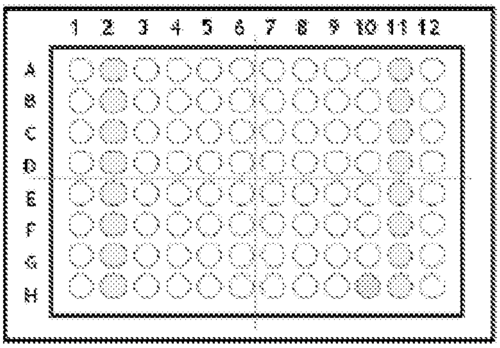


Figure 2D

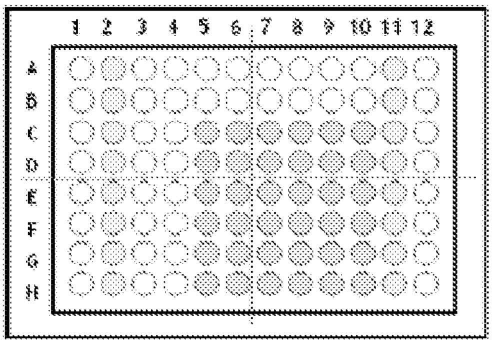

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Littérature non-brevet citée dans la description


- **GRARE et al.** *J. Antimicrob. Chemother.,* 2007, vol. 60, 575-581
- **GRARE et al.** *Clin. Microbiol. Infect.,* 2010, vol. 16, 432-438
- **GRARE et al.** *Pathologie Biologie,* 2010, vol. 58, 46-51
- **MOURER et al.** *Bioorganic & Médicinal Chemistry Letter,* 2006, vol. 16, 2960-2963
- **BERGE et al.** Pharmaceutical Salts. *Journal of Pharmaceutical Science,* 1977, vol. 66, 1-19